(19) Europäisches Patentamt
European Patent Office
Office européen des brevets

(11) **EP 4 520 825 A1**

(12) **EUROPEAN PATENT APPLICATION**
published in accordance with Art. 153(4) EPC

(43) Date of publication:
**12.03.2025 Bulletin 2025/11**

(21) Application number: **23799444.7**

(22) Date of filing: **20.04.2023**

(51) International Patent Classification (IPC):
***C12N 15/113*** *(2010.01)* ***C12N 15/10*** *(2006.01)*
***C12N 15/63*** *(2006.01)* ***C12P 19/34*** *(2006.01)*

(52) Cooperative Patent Classification (CPC):
**C12N 15/10; C12N 15/113; C12N 15/63; C12P 19/34**

(86) International application number:
**PCT/JP2023/015828**

(87) International publication number:
**WO 2023/214512 (09.11.2023 Gazette 2023/45)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **06.05.2022 JP 2022076674**

(71) Applicants:
- **Josho Gakuen Educational Foundation**
  **Osaka 535-8585 (JP)**
- **National University Corporation**
  **Hokkaido University**
  **Hokkaido 060-0808 (JP)**

(72) Inventors:
- **YOSHIMOTO Rei**
  **Hirakata-shi, Osaka 573-0101 (JP)**
- **NAKAGAWA Shinichi**
  **Sapporo-shi, Hokkaido 060-0808 (JP)**

(74) Representative: **Vossius & Partner**
**Patentanwälte Rechtsanwälte mbB**
**Siebertstrasse 3**
**81675 München (DE)**

Remarks:
The complete document including Reference Table(s) and the Sequence Listing(s) can be downloaded from the EPO website

# (54) ARTIFICIAL RNA MOLECULE

(57) It is an object of the present invention to provide a novel technique of regulating mRNA splicing. Provided is an artificial RNA molecule comprising (a) a polynucleotide potentially having a secondary structure represented by the formula (I), or a polynucleotide in which 1 to 3 bases are substituted, deleted, or added among 7 bases on the 3' side of the polynucleotide potentially having the secondary structure represented by the formula (I); and (b) a pre-mRNA targeting polynucleotide comprising a sequence complementary to a target sequence that is a portion of a pre-mRNA; wherein the (a) and the (b) are arranged from the 5' side to the 3' side in this order.

FIG. 1

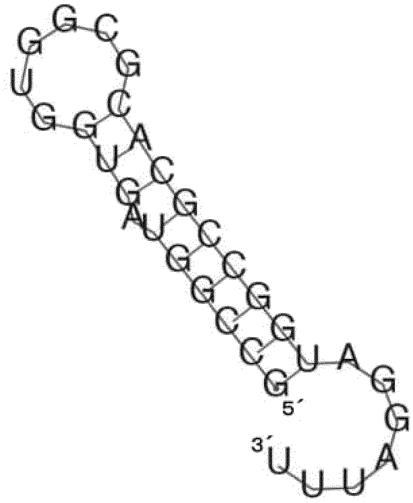

EP 4 520 825 A1

## Description

### TECHNICAL FIELD

The present invention relates to a novel artificial RNA molecule, in particular an artificial RNA molecule that regulates splicing of mRNA.

### BACKGROUND ART

4.5SH RNA is a non-coding RNA specific to Rodentia and Myomorpha animals (Myodonta Clade), and its base sequence is identified (Non-Patent Document 1). However, functions of 4.5SH RNA have not been elucidated.
On the other hand, gene therapy has been attracting attention in recent years. As gene therapy, for example, nucleic acid drugs and techniques that adapt genome editing with CRISPR/Cas9 are known.
As nucleic acid drugs, for example, antisense nucleic acid drugs that treat neurological and muscular diseases by regulating mRNA splicing of disease-causing genes are available on the market (for example, Patent Document 1).
As techniques that adapt genome editing with CRISPR/Cas9, for example, a technique of transplanting patient-derived stem cells, in which expressions of disease-causing genes, specifically β-globin genes that have disease-causing mutations, etc., are reduced and expressions of the corresponding normal genes are increased, into a patient, and the like have been developed (for example, Patent Document 2).

### PRIOR ART DOCUMENT

#### Patent Document

Patent Document 1: JP 2014-54250 A
Patent Document 2: JP 2021-166514 A

#### Non-Patent Document

Non-Patent Document 1: Harada, F and Kato, N. (1980) NAR., 8:1273-J285

### SUMMARY OF THE INVENTION

#### PROBLEMS TO BE SOLVED BY THE INVENTION

However, antisense nucleic acid drugs are generally about 14 to 25 bases in length, and therefore they require screening to find sites that are effective in regulating mRNA splicing from target genes, making their developments to be time-consuming and costly. Moreover, because of their short half-life, the drug efficacy cannot be maintained unless it is administered repeatedly every few weeks to months, which places a significant burden on the medical economy.
One of methods for solving this problem is to use a technique that adapts genome editing with CRISPR/Cas9. The technique that adapts genome editing acts directly on a genome, allowing the effect to last for a long time.
As an expression vector for a CRISPR/Cas9 system, an adeno-associated virus vector (AAV) is often used from the viewpoints of safety, etc. However, a Cas9 gene has a large molecular weight, making it difficult to load into the AAV.
In light of such a situation as described above, there is a need for a novel technique that can control gene expression different from genome editing with antisense nucleic acids or CRISPR/Cas9.
Therefore, it is an object of the present invention to provide a novel technique of regulating mRNA splicing.

#### MEANS TO SOLVE THE PROBLEM

As a result of intensive studies, the present inventors have found that a non-coding RNA specific to Rodentia and Myomorpha animals is involved in mRNA splicing. Then, after further studies, the present inventors have found that the above-described problems can be solved so that a characteristic secondary structure of a specific sequence of the above-described non-coding RNA specific to Rodentia and Myomorpha animals serves to attract a splicing control factor and stabilize an RNA molecule, and by using an artificial RNA molecule in which this secondary structure is combined with a sequence complementary to a targeted pre-mRNA, mRNA splicing can be regulated, and completed the present invention.

That is, the present invention relates to:

[1] An artificial RNA molecule comprising:

(a) a polynucleotide potentially having a secondary structure represented by the following formula (I), or a polynucleotide in which 1 to 3 bases are substituted, deleted, or added among 7 bases on the 3' side of the polynucleotide potentially having the secondary structure represented by the following formula (I):

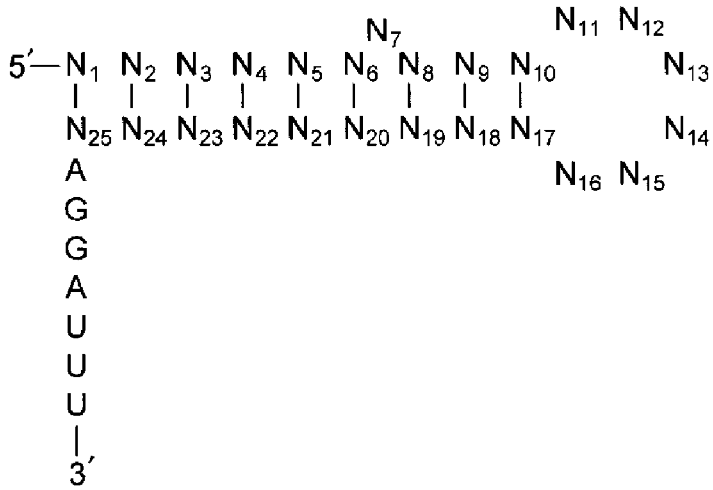

Formula（ I ）

(wherein, $N_1$ to $N_{25}$ each independently represents A, C, G, or U, and $N_1$ and $N_{25}$, $N_2$ and $N_{24}$, $N_3$ and $N_2$s, $N_4$ and $N_{22}$, $N_5$ and $N_{21}$, $N_6$ and $N_{20}$, $N_8$ and $N_{19}$, $N_9$ and $N_{18}$, and $N_{10}$ and $N_{17}$, respectively, form a base pair.); and
(b) a pre-mRNA targeting polynucleotide comprising a sequence complementary to a target sequence that is a portion of a pre-mRNA; wherein said (a) and (b) are arranged from the 5' side to the 3' side in this order,

[2] The artificial RNA molecule of [1] above, wherein the artificial RNA molecule regulates splicing of the pre-mRNA targeted by said (b),
[3] The artificial RNA molecule of [1] or [2] above, wherein a base sequence of said (a) is the sequence set forth in SEQ ID NO: 2 or a sequence having a sequence identity of 80% or more with the sequence shown in SEQ ID NO: 2,
[4] The artificial RNA molecule of any one of [1] to [3] above, wherein a length of said (b) is 15 to 300 bases, more preferably 20 to 60 bases,
[5] The artificial RNA molecule of any one of [1] to [4] above, wherein the target sequence is an exon sequence,
[6] The artificial RNA molecule of any one of [1] to [5] above, comprising:
(c) a polynucleotide comprising a sequence that contributes to terminal stability,
wherein said (c) is arranged on the 3' side of said (b),
[7] The artificial RNA molecule of [6] above, wherein said (c) is a polynucleotide comprising a transcription termination signal sequence recognized by an RNA pol III enzyme,
[8] The artificial RNA molecule of any one of [1] to [7] above, wherein said (a) is a polynucleotide binding to a protein that controls mRNA splicing,
[9] The artificial RNA molecule of any one of [1] to [8] above, wherein the target sequence is a partial sequence of a gene selected from an FAS gene, a dystrophin gene, and a fukutin gene,
[10] The artificial RNA molecule of any one of [1] to [9] above, wherein the target sequence is a partial sequence of an MAPT gene,
[11] An expression vector comprising a DNA sequence encoding an artificial RNA molecule of any one of [1] to [10] above,
[12] A method of regulating mRNA splicing, comprising:

(A) preparing an artificial RNA molecule comprising:

(a) a polynucleotide potentially having a secondary structure represented by the formula (I), or a polynucleotide in which 1 to 3 bases are substituted, deleted, or added among 7 bases on the 3' side of the polynucleotide potentially having the secondary structure represented by the formula (I); and
(b) a pre-mRNA targeting polynucleotide comprising a sequence complementary to a target sequence that is

a portion of a pe-mRNA;

wherein said (a) and (b) are arranged from the 5' side to the 3' side in this order; and
(B) contacting the artificial RNA molecule prepared in the step (A) with a pre-mRNA to form a complementary strand between the artificial RNA molecule and the pre-mRNA, thereby regulating mRNA splicing,

[13] The method of [12] above, wherein the regulation of mRNA splicing is caused by exon skipping,
[14] The method of [12] or [13] above, wherein the base sequence of said (a) is the sequence set forth in SEQ ID NO: 2 or the sequence having the sequence identity of 80% or more with the sequence shown in SEQ ID NO: 2,
[15] The method of any one of [12] to [14] above, wherein a length of said (b) is 15 to 300 bases, more preferably 20 to 60 bases,
[16] The method of any one of [12] to [15] above, wherein the target sequence is an exon sequence,
[17] The method of any one of [12] to [16] above, wherein the artificial RNA molecule comprises a polynucleotide comprising a transcription termination signal sequence recognized by an RNA pol III enzyme, the polynucleotide being arranged on the 3' side of said (b),
[18] A method of producing a mature mRNA, the method comprising the steps of:

(A) preparing an artificial RNA molecule comprising:

(a) a polynucleotide potentially having a secondary structure represented by the formula (I), or a polynucleotide in which 1 to 3 bases are substituted, deleted, or added among 7 bases on the 3' side of the polynucleotide potentially having the secondary structure represented by the formula (I); and
(b) a pre-mRNA targeting polynucleotide comprising a sequence complementary to a target sequence that is a portion of an pre-mRNA;

wherein said (a) and (b) are arranged from the 5' side to the 3' side in this order:

(B') contacting the artificial RNA molecule prepared in the step (A) with the pre-mRNA to form a complementary strand between the artificial RNA molecule and the pre-mRNA, and
(C) regulating mRNA splicing by the step (B') to produce a mature mRNA,

[19] The method of [18] above, wherein the regulation of mRNA splicing is caused by exon skipping,
[20] The method of [18] or [19] above, wherein the base sequence of said (a) is the sequence shown in SEQ ID NO: 2 or the sequence having the sequence identity of 80% or more with the sequence shown in SEQ ID NO: 2,
[21] The method of any one of [18] to [20] above, wherein a length of said (b) is 15 to 300 bases, more preferably 20 to 60 bases,
[22] The method of any one of [18] to [21] above, wherein the target sequence is an exon sequence, and
[23] The method of any one of [18] to [22] above, wherein the artificial RNA molecule comprises a polynucleotide comprising a transcription termination signal sequence recognized by an RNA pol III enzyme, the polynucleotide being arranged on the 3' side of said (b).

## EFFECTS OF THE INVENTION

According to the present invention, provided is a novel technique of regulating mRNA splicing that differs from conventional techniques by an artificial RNA molecule that combines a characteristic secondary structure with a sequence complementary to a target pre-mRNA, and a method using the artificial RNA molecule.

## BRIEF DESCRIPTION OF THE DRAWINGS

FIG. 1 is a schematic view showing a part of a secondary structure of 4.5SH RNA predicted by a secondary structure prediction program RNAfold.
FIG. 2 is an electrophoretic view obtained by performing RT-PCR using primers that specifically amplify each of three genes carrying asSINEB1 in wild-type and 4.5SH knockout mice. From left to right, the results are for Smchd1, Slc25a40, and Smg6. The markers shown in the schematic view on the left show mRNAs comprising a de novo exon in grey for the upper stage and mRNAs not comprising a de novo exon for the lower stage. The exon in question is represented in grey, and both sides thereof are represented in black and white.

FIG. 3 is a schematic view of a reporter carrying a U6 promoter and a mouse 4.5SH sequence. The U6 promoter sequence and the 4.5SH sequence are in turn arranged from the 5' side of the reporter.

FIG. 4 is an electrophoretic view showing results obtained by confirming by Northern blotting that 4.5SH RNA is expressed in a human cell by introducing and expressing the promoter shown in FIG. 3.

FIG. 5 is an electrophoretic view obtained when minigenes, each incorporating two genes having asSINEB1, were introduced into human 4.5SH-expressing cells, and RT-PCR was performed using primers that specifically amplify each gene. From left to right, the results are for Slc25a40 and Smg6. The markers shown in the schematic view on the left show mRNAs comprising a de novo exon in grey for the upper stage and mRNAs not comprising a de novo exon for the lower stage.

FIG. 6 shows bar graphs indicating skipping efficiencies determined from the electrophoretic view of FIG. 5. The result for SIc25a40 is shown on the left, and the result for Smg6 is shown on the right.

FIG. 7 shows a part of each of DNA base sequences of plasmids expressing, from the top, a wild-type 4.5SH (WT), a wild-type SIc25a40 (WT), a mutant-type 4.5SH (MUT), and a mutant-type SIc25a40 (MUT). Parts that differ in base between WT and MUT are shown in grey.

FIG. 8 is an electrophoretic view obtained when a mutant-type plasmid and a wild-type plasmid were introduced into human cells, and RT-PCR was performed using primers that specifically amplify each of them.

FIG. 9 shows a bar graph indicating skipping efficiencies determined from the electrophoretic view of FIG. 8. From the left, FIG. 9 show a wild-type SIc25a40 alone, two wild types, a mutant-type SIc25a40 and a wild-type 4.5SH, a wild-type SIc25a40 and a mutant-type 4.5SH, and two mutant types.

FIG. 10 shows exon skipping efficiencies of a B1-modified 4.5SH (SL-B1-4.5SH). From the top, FIG. 10 shows an electrophoretic view obtained using BioAnalyzer, an electrophoretic view showing results of confirming RNA expression by Northern blotting, and a bar graph showing exon skipping efficiencies. The markers shown in the schematic view on the left of the electrophoretic view obtained using BioAnalyzer show mRNAs comprising a de novo Slc25a40 exon (exon 8.5) for the upper stage and mRNAs not comprising SIc25a40 exon 8.5 for the lower stage.

FIG. 11 is an electrophoretic view obtained when a minigene for detecting exon skipping of an FAS gene was co-expressed with a wild-type 4.5SH or an FAS-modified 4.5SH.

FIG. 12 is an electrophoretic view obtained when a minigene comprising an Slc25a40 exon 8.5 was co-expressed with a wild-type 4.5SH or an FAS-modified 4.5SH.

FIG. 13 show a bar graph indicating skipping efficiencies determined from the electrophoretic views of FIGS. 11 and 12.

FIG. 14 is a schematic view of a target sequence of a DMD gene-modified 4.5SH.

FIG. 15 is an electrophoretic view obtained when a minigene for detecting exon skipping of a DMD gene was co-expressed with a DMD gene-modified 4.5SH. The markers shown in the schematic view on the left show mRNAs comprising a DMD exon 53 in grey for the upper stage and mRNAs not comprising a DMD exon 53 for the lower stage.

FIG. 16 is an electrophoretic view by BioAnalyzer obtained when a minigene for detecting exon skipping of a DMD gene was co-expressed with a DMD gene-modified 4.5SH. The schematic view on the top is a schematic view describing targeting of the DMD gene-modified 4.5SH to a DMD exon 53. The markers shown in the schematic view on the left show mRNAs comprising a DMD exon 53 for the upper stage and mRNAs not comprising a DMD exon 53 for the lower stage.

FIG. 17 is a schematic view of a target sequence of a DMD gene-modified 4.5SH.

FIG. 18 shows results obtained when a minigene for detecting exon skipping of a DMD gene was co-expressed with a DMD gene-modified 4.5SH. From the top, FIG. 18 shows an electrophoretic view obtained using BioAnalyzer, an electrophoretic view showing results of confirming RNA expression by Northern blotting, and a bar graph showing exon skipping efficiencies. The markers shown in the schematic view on the left of the electrophoretic view obtained using BioAnalyzer show mRNAs comprising a DMD exon 53 for the upper stage and mRNAs not comprising a DMD exon 53 for the lower stage.

FIG. 19 shows exon skipping efficiencies of an MAPT gene-modified 4.5SH. From the top, FIG. 19 shows a schematic view describing targeting of the MAPT gene-modified 4.5SH to an MAPT exon 10, an electrophoretic view obtained using BioAnalyzer, an electrophoretic view showing results of confirming RNA expression by Northern blotting, and a bar graph showing exon skipping efficiencies. The markers shown in the schematic view on the left of the electrophoretic view obtained using BioAnalyzer show mRNAs comprising an MARTexon 10 for the upper stage and mRNAs not comprising an MART exon 10 for the lower stage.

## EMBODIMENT FOR CARRYING OUT THE INVENTION

Although it not intended to be bound by theory, for example, the following can be considered as a mechanism in which effects of the present invention are exhibited.

In the artificial RNA molecule of the present invention, (a) a polynucleotide potentially having a secondary

structure represented by the formula (I), or a polynucleotide in which 1 to 3 bases are substituted, deleted, or added among 7 bases on the 3' side of the polynucleotide potentially having the secondary structure represented by the formula (I) (polynucleotide (a)) forms a strong stem-loop structure, to which a splicing control factor binds, and by (b) a pre-mRNA targeting polynucleotide comprising a sequence complementary to a target sequence that is a part of a pre-mRNA (polynucleotide (b)), binds to the target sequence in the pre-mRNA. Then, the splicing control factor bound to the polynucleotide (a) acts suppressively or promotionally on splicing reaction, so that a part comprising the target sequence, for example an exon, is skipped or included during mRNA splicing.

<Definition>

In the present specification, an "artificial RNA molecule" is an expression to mean an RNA molecule that does not exist in nature, which is an artificially produced RNA molecule that corresponds to the artificial RNA molecule comprising the polynucleotide (a) and the polynucleotide (b) of the present invention, though it shall not be included in the artificial RNA molecule of the present invention if any sequence of the entire molecule exists in nature.

In the present specification, a "pre-mRNA" refers to RNA that is transcribed in the cell nucleus by an RNA polymerase enzyme using DNA as a template prior to being subjected to mRNA splicing.

In the present specification, "mRNA splicing" refers to a step by which an intron is removed from a pre-mRNA and a mature mRNA is created from the pre-mRNA. Moreover, the same applies to a case where the term is simply referred to as "splicing".

In the present specification, an "exon" refers to a sequence contained in mature mRNA. A person skilled in the art can obtain an annotated exon sequence from publicly accessible databases such as, for example, Ensembl, National Center for Bio-technique Information (NCBI), GenBank, or other NCBI databases, using a computer implemented with a software. Specifically, for example, regarding human genes, a person skilled in the art can select a gene from the following NCBI search query: http://www.ncbi.nlm.nih.gov/gene/?term=Homo+sapiens[Orgn]. Alternatively, a person skilled in the art can view a genome at the Ensembl database (http://www.ensembl.org) and obtain information regarding an exon.

In the present specification, an "intron" refers to a sequence not contained in a mature mRNA. Similarly with the case of the "exon" above, a person skilled in the art can obtain an annotated intron sequence from publicly accessible databases such as NCBI, GenBank, or other NCBI databases, using a computer implemented with a software.

In the present specification, "alternative splicing" refers to splicing conducted with a specific exon removed.

In the present specification, "base pairing" incudes not only so-called Watson-Crick base pairing by which adenine (A) and thymine (T) (or uracil (U)) hydrogen-bond with each other and guanine (G) and cytosine (C) hydrogen-bond with each other, of a nucleotide, but also wobble base pairing between G and U, inosine (I) and U, and I and A, etc.

In the present specification, "exonization" refers to the inclusion of a sequence that is originally an intron in a mature mRNA as a result of regulated mRNA splicing.

In the present specification, a "de novo exon" refers to a sequence that was originally an intron and generated by exonization.

In the present specification, a "stem-loop structure" refers to a secondary structure formed within a single nucleic acid molecule, which is formed by a nucleotide sequence that is folded by the nucleic acid molecule itself. The stem-loop structure consists of a stem region, created by a base sequence located on the 3' terminal side and a base sequence located on the 5' terminal side contained in the same molecule forming base pairing, and a loop structure region formed by a base sequence located between the base sequence located on the 3' terminal side and the base sequence located on the 5' terminal side that constitute the stem region. A person skilled in the art can verify formation and strength of a stem-loop structure by a secondary structure prediction program such as RNA Fold (http://rna.tbi.univie.ac.at/cgi-bin/RNAWebSuite/RNAfold.cgi) and the like, as a thermodynamic simulation, and by RNA footprint assay, nuclear magnetic resonance (NMR) and crystal structure analysis of a nucleic acid molecule, as an experimental technique.

The strength of the stem-loop structure can be evaluated by the minimum free energy (MFE) that is an index showing stability of the secondary structure. A smaller MFE value indicates a more stable secondary structure and a stronger stem-loop structure. MFE can be calculated using the secondary structure prediction program such as RNA Fold (http://rna.tbi.univie.ac.at/cgi-bin/RNAWebSuite/RNAfold.cgi) and the like.

In the present specification, "exon skipping" refers to a mode of alternative splicing in which a mature mRNA lacks a specific exon that is originally present.

In the present specification, an "exon skipping efficiency" can be calculated from a polynucleotide amount (X) and its nucleotide length ($L_x$) of a band skipped by an exon and a polynucleotide amount (Y) and its nucleotide length ($L_y$) of a band skipped by the exon, by the following equation:

$$\text{Skipping efficiency (\%)} = \{X/L_x \div (X/L_x + Y/L_y)\} \times 100$$

In a first embodiment, the present invention provides an artificial RNA molecule (also referred to as an artificial RNA molecule of the present invention) comprising:

(a) a polynucleotide potentially having a secondary structure represented by the above-described formula (I), or a polynucleotide in which 1 to 3 bases are substituted, deleted, or added among 7 bases on the 3' side of the polynucleotide potentially having the secondary structure represented by the above-described formula (I); and
(b) a pre-mRNA targeting polynucleotide comprising a sequence complementary to a target sequence that is a portion of an pre-mRNA; wherein said (a) and (b) are arranged from the 5' side to the 3' side in this order.

Hereinafter, in the present invention, (a) a polynucleotide potentially having a secondary structure represented by the formula (I), or a polynucleotide in which 1 to 3 bases are substituted, deleted, or added among 7 bases on the 3' side of the polynucleotide potentially having the secondary structure represented by the formula (I) may be simply referred to as "polynucleotide (a)", (b) a pre-mRNA targeting polynucleotide comprising a sequence complementary to a target sequence that is a part of a pre-mRNA may be simply referred to as "polynucleotide (b)", and (c) a polynucleotide comprising a sequence that contributes to terminal stability, which will be mentioned later, may be simply referred to as "polynucleotide (c)".

<Polynucleotide (a)>

Polynucleotide (a) is a polynucleotide potentially having a secondary structure represented by the following formula (I), or a polynucleotide in which 1 to 3 bases are substituted, deleted, or added among 7 bases on the 3' side of the polynucleotide potentially having the secondary structure represented by the following formula (I).

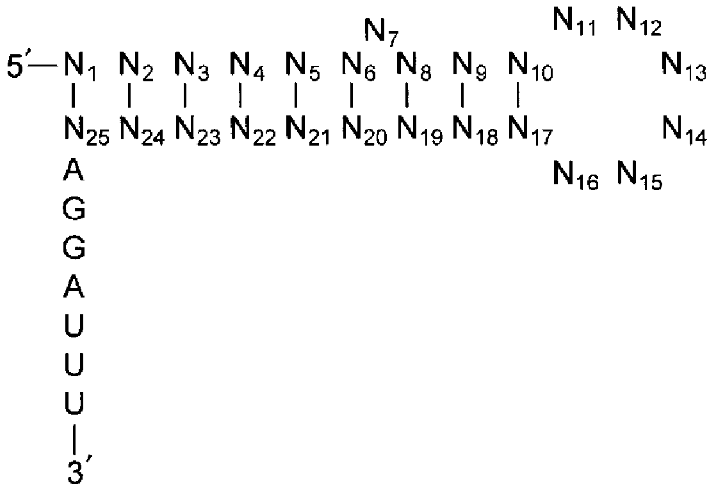


Formula (I)

(wherein, $N_1$ to $N_{25}$ each independently represents A, C, G, or U, and $N_1$ and $N_2$s, $N_2$ and $N_{24}$, $N_3$ and $N_2$s, $N_4$ and $N_{22}$, $N_5$ and $N_{21}$, $N_6$ and $N_{20}$, $N_8$ and $N_{19}$, $N_9$ and $N_{18}$, and $N_{10}$ and $N_{17}$, respectively, form a base pair.).

The polynucleotide (a) forms a strong stem-loop structure relating to $N_1$ to $N_2$s, as shown in the formula (I), as a secondary structure that exists stable under a physiological condition.

In order for $N_1$ to $N_{25}$ to form a strong stem-loop structure through base pairing within a single RNA molecule, it is preferable that $N_1$ to $N_{10}$ and $N_{17}$ to $N_{25}$ are rich in guanine (G) and cytosine (C) or G and uracil (U), respectively. Nine base pairings formed between $N_1$ and $N_2$s, $N_2$ and $N_{24}$, $N_3$ and $N_2$s, $N_4$ and $N_{22}$, $N_5$ and $N_{21}$, $N_6$ and $N_{20}$, $N_8$ and $N_{19}$, $N_9$ and $N_{18}$, and $N_{10}$ and $N_{17}$ are preferably formed by G and C or G and U. Of the nine base pairings, the number of base pairings formed by G and C or G and U is preferably 5 to 9, more preferably 6 to 9, further preferably 7 to 9, further preferably 8 to 9, further preferably 5 to 8, further preferably 6 to 8, particularly preferably 7 to 8, most preferably 8. $N_{11}$ to $N_{16}$ are preferably RYRRYR to form a loop structure. Here, R represents a purine base (A or G) and Y represents a pyrimidine base (C or U).

The seven bases on the 3' side from $N_{25}$ as shown in the formula (I) do not necessarily have to be bases that form a stem-loop structure, and one to three bases may be substituted, deleted, or added. Substitution, deletion, or addition of a base may be performed for any of the seven bases shown in the formula (I), preferably one or two bases, more preferably one base. In the case of substitution, it is preferable that purine bases are substituted with each other and pyrimidine bases are substituted with each other. In the case of addition, it is particularly preferable that a base is added to U at the 3' terminal.

A sequence (primary structure) of the polynucleotide (a) is not particularly limited as long as it is a base sequence that potentially has the secondary structure shown in the formula (I), but it preferably includes the sequence shown in SEQ

ID NO: 2 or a sequence having 80% or more sequence identity to the sequence shown in SEQ ID NO: 2. The sequence shown in SEQ ID NO: 2 is rich in G and C and can form a strong stem-loop structure shown in the formula (I) as will be mentioned later (FIG. 1).

The polynucleotide (a) has more preferably 85% or more, further preferably 90% or more, particularly preferably 92% or more, most preferably 95% or more sequence identity to the sequence shown in SEQ ID NO: 2.

The MFE of the stem-loop structure of the polynucleotide (a) is preferably -10.00 kcal/mol or less, more preferably -11.00 kcal/mol or less, further preferably -12.00 kcal/mol or less, particularly preferably -12.80 kcal/mol or less, most preferably -12.90 kcal/mol or less. It is considered that, when the strength of the stem-loop structure is within the above-described ranges, the RNA molecule is stabilized.

The MFE of the stem-loop structure can vary depending on the number of base pairings in the stem region (length of the complementary strand), the number of mismatched or bulged bases, the number of bases forming a loop structure, and the like.

The polynucleotide (a) comprises a stem-loop structure and subsequent bases on the 3' side, as mentioned above, which is thereby considered to contribute to stabilization of the artificial RNA molecule. Moreover, the polynucleotide (a) is considered to play a role in attracting splicing control factors.

In the present specification, a "splicing control factor" is an expression to mean all factors that control mRNA splicing present in a living body, and specifically includes proteins and nucleic acids that control mRNA splicing, as well as complexes of protein and RNA.

The polynucleotide (a) is considered to have functions of attracting a splicing control factor and binding to the splicing control factor. The splicing control factor that binds to the polynucleotide (a) determines what kind of regulation the artificial RNA molecule of the present invention performs in mRNA splicing, that is, what kind of alternative splicing is caused.

More specifically, examples of the splicing control factor that binds to the polynucleotide (a) include proteins that constitute a small nuclear RNA, U2 complex, binding proteins thereof, and the like. Further specifically, they include SF3b1, U2AF2, Nono, Sfpq, Hnrnpf, and the like. Moreover, it is inferred from results of mass spectrometry that Hnrnpm, Hnrnpa1, Hnrnpa2b1, Ddx5, Hnrnpa3, Fus, Hnrnpa0, Pabpc1, Snrpa, Dhx9, Myef2, Ddx17, Hnrnph1, Prpf8, Rps3a, Rbm14, and the like bind.

As functions of these splicing control factors, a function to suppress a sequence such as a splice donor sequence or a splice acceptor sequence necessary to enable splicing reaction or a function to suppress an exon inclusion signal, a function to involve in accurate recognition of an intron sequence, etc. are known.

As specific examples of the polynucleotide (a) include a sequence (SEQ ID NO: 2) discovered in 4.5SH RNA (hereinafter also referred to as 4.5SH) that is a non-coding RNA specific to Rodentia and Myomorpha animals. In 4.5SH, the sequence shown in SEQ ID NO: 2 was found to be an essential sequence for forming a stem-loop structure as a secondary structure that exists stable under a physiological condition, attracting a splicing control factor, and regulating mRNA splicing of a pre-mRNA sequence targeted by 4.5SH. The sequence targeted by 4.5SH and its regulation of mRNA splicing will be mentioned later. Besides, 4.5SH is transcribed from DNA by an RNA polymerase III enzyme (RNA pol III enzyme).

A sequence corresponding to the polynucleotide (a) in 4.5SH does not form a complementary strand with a target sequence, and is therefore considered to attract an mRNA splicing control factor and inhibit accurate recognition of an intron. Moreover, the sequence shown in SEQ ID NO: 2 is considered to contribute to stability of the entire 4.5SH since it forms a strong stem-loop structure as shown in the formula (I) and FIG. 1.

The polynucleotide (a) of the present invention will be described below with reference to the drawing, taking as an example the sequence shown in SEQ ID NO: 2 that is a specific example thereof.

FIG. 1 shows a secondary structure of a part of the sequence shown in SEQ ID NO: 2 in 4.5SH as predicted by RNA Fold (http://rna.tbi.univie.ac.at/cgi-bin/RNAWebSuite/RNAfold.cgi). In FIG. 1, G that is a terminal of the stem-loop structure corresponds to a base on the 5' terminal in the above-described formula (I).

In the present specification, "the Xth base" means the Xth base counted from the 5' side. In FIG. 1 , the sequence shown in SEQ ID NO: 2 in 4.5SH forms a stem region by base pairing between the 1st G and the 25th U, between the 2nd C and the 24th G, between the 3rd C and the 23rd G, between the 4th G and the 22nd C, between the 5th G and the 21st C, between the 6th U and the 20th G, between the 8th G and the 19th C, between the 9th U and the 18th A, and between the 10th G and the 17th C. The 11th to 16th bases are bases that have no partners for forming base pairing and form a loop structure.

In FIG. 1, the 26th A to the 32nd U are not bases that form a stem-loop structure. Moreover, in 4.5SH, the bases from the 26th A to the 32nd U in the sequence shown in SEQ ID NO: 2 are not bases that form a stem-loop structure, nor bases that form a complementary strand with a target sequence of 4.5SH that will be mentioned later.

<Polynucleotide (b)>

The polynucleotide (b) is a pre-mRNA targeting polynucleotide that comprises a sequence complementary to a target sequence that is a partial sequence of a pre-mRNA. The polynucleotide (b) can be designed to have any sequence depending on a target sequence.

The polynucleotide (b) comprises a sequence complementary to a target sequence that is a partial sequence of a pre-mRNA. That is, the polynucleotide (b) forms a complementary strand with the target sequence. To form a complementary strand, two nucleic acid molecules do not need to be completely (100%) complementary.

A complementary sequence refers to a sequence for two nucleic acid molecules to be complementary to each other through base pairing. For example, it may be a sequence having 80 to 100% complementarity (e.g., a sequence in which 4 or 5 of 5 bases are complementary), or a sequence having 90 to 100% identity (e.g., a sequence in which 9 or 10 of 10 bases are complementary).

A complementarity of the base sequence to the polynucleotide (b) and the target sequence is preferably 80% or more, more preferably 85% or more, further preferably 90% or more.

A base length of the polynucleotide (b) is preferably 15 bases or more, more preferably 20 bases or more, further preferably 40 bases or more, particularly preferably 50 bases or more, from the viewpoints of stability of the complementary strand to the target sequence and specificity of the complementary strand to the target sequence. Moreover, the base length of the polynucleotide (b) is preferably 300 bases or less, more preferably 200 bases or less, further preferably 100 bases or less, particularly preferably 80 bases or less, most preferably 60 bases or less, from the viewpoint of ease of synthesis of the artificial RNA molecule.

The stability of the complementary strand between the polynucleotide (b) and the target sequence can vary depending not only on complementarity of the base sequences described above, but also on the number of hydrogen bonds between the bases (for example, the pair of G and C has more hydrogen bonds than the pair of A and U) and a position of a non-complementary sequence (whether it is in the middle or at the terminal of the complementary strand, etc.).

The target sequence of the polynucleotide (b) is not particularly limited as long as it is a partial base sequence of a pre-mRNA that is a target for regulating mRNA splicing, but is preferably an exon sequence relating to an exon whose presence or absence in a mature mRNA changes a function of a protein encoded by mRNA. In this case, the exon sequence may be the entire base sequence of the exon or a part of the base sequence of the exon, depending on a length of a targeted exon. Moreover, the target sequence of the polynucleotide (b) may include, in addition to the exon sequence, a part of an intron sequence located within 300 bases upstream (5' side) or downstream (3' side) of the exon, or may be only a part of the intron sequence not including the exon sequence.

The polynucleotide (b) may comprise a plurality of sequences each complementary to a plurality of target sequences. Such a plurality of target sequences are, but not limited to, preferably selected from a region in a range including the same exon and 300 bases upstream or downstream thereof.

When the target sequence is an exon sequence and the exon sequence has a length equal to or longer than an average length of exons (about 150 bases), the polynucleotide (b) preferably comprises a plurality of sequences each complementary to a plurality of target sequences. In such a case, the base length of the polynucleotide (b) is preferably 60 bases or more, more preferably 90 bases or more, further preferably 100 bases or more. Moreover, it is preferably 400 bases or less, more preferably 300 bases or less.

<Polynucleotide (c)>

The artificial RNA molecule of the present invention preferably comprises, in addition to the above-mentioned polynucleotide (a) and polynucleotide (b), a polynucleotide comprising a sequence that contributes to terminal stability (polynucleotide (c)), and this polynucleotide (c) is preferably arranged on the 3' side of the polynucleotide (b).

The polynucleotide (c) is preferably a polynucleotide comprising a transcription termination signal sequence recognized by an RNA pol III enzyme in order for the artificial RNA molecule of the present invention to function as a non-coding RNA. The transcription termination signal sequence recognized by the RNA pol III enzyme is a sequence in which seven or more uracils (U) are linked.

The transcription termination signal sequence recognized by the RNA pol III enzyme not only functions as a signal to terminate transcription when the non-coding RNA is transcribed from DNA, but may also contribute to stabilization of the entire non-coding RNA. Besides the transcription termination signal sequence recognized by the RNA pol III enzyme, a structure of the 3' terminal of the non-coding RNA may also contribute to stabilization of the entire non-coding RNA (see Trends in Genetics, 2007 Dec; vol 23: 614-622, Biochim Biophys Acta. 2013 Mar; 1829(3-4): 318-330, etc.). In the structure of the 3' terminal of the non-coding RNA, a sequence of miRNA or a self-cleaving ribozyme may be encoded upstream of the transcription termination signal sequence recognized by the RNA pol III enzyme (see Trends in Genetics, 2007 Dec; vol 23: 614-622, Biochim Biophys Acta. 2013 Mar; 1829(3-4): 318-330, etc.).

<Artificial RNA molecules>

The artificial RNA molecule of the present invention regulates mRNA splicing of a pre-mRNA targeted by the polynucleotide (b). Examples of the regulation of mRNA splicing include modifications of intron retention and alternative splicing, and among them, modification of alternative splicing is preferable. Examples of modification of alternative splicing include inclusion induction of an exon or skipping induction of the exon (exon skipping). Among them, skipping induction of the exon is preferable since the function of 4.5SH is to suppress de novo exonization due to exon skipping.

In the present invention, at least one polynucleotide (a) and at least one polynucleotide (b) are arranged from the 5' side to the 3' side in this order. Moreover, when the artificial RNA molecule comprises a polynucleotide (c), the polynucleotide (c) is arranged on the 3' side of the polynucleotide (a) and the polynucleotide (b).

Two or more of each of the polynucleotide (a), the polynucleotide (b), and the polynucleotide(c) may be arranged, or one of each may be arranged. Since the polynucleotide (a) is a sequence essential for regulating mRNA splicing, the effects of the present invention may be further exhibited by arranging two or more polynucleotides (a). When two or more polynucleotides (a) are arranged, at least two structures represented by the formula (I) may be arranged via a multi-branched loop. Moreover, even if three or more polynucleotides (a) are arranged, it is difficult to expect any further increase in effect, and from the viewpoint of stability of the artificial RNA molecule, it is preferable to arrange three or less polynucleotides (a). When two or more polynucleotides (a) are arranged, the second polynucleotide (a) is preferably arranged on the 3' side of the polynucleotide (b).

A spacer may or may not be present among the polynucleotide (a), the polynucleotide (b), and the polynucleotide (c), respectively. When a spacer is present, a base length of the spacer can be, for example, 200 bases or less, 150 bases or less, 100 bases or less, 50 bases or less, 40 bases or less, or 30 bases or less.

The nucleic acid in the artificial RNA molecules of the present invention may be a natural or modified nucleic acid. The modified nucleic acid may include, for example, nucleic acid in which an OH group at 2'-position of ribose is substituted with an F group or an OMe group, phosphorothioate nucleic acid, morpholino nucleic acid, a bridged nucleic acid (LNA), and the like, from the viewpoints of increasing resistance to nuclease and hydrolysis, etc. Moreover, it may include pseudouridine ($\Psi$), N1-methylpseudouridine (N1m$\Psi$), and the like, instead of uridine (U), from the viewpoints of reducing cytotoxicity, etc. The modified nucleic acid can be located at all or a part of positions, and if it is located at a part of positions, it can be located on a random position in any proportion.

<5' terminal>

A structure commonly used in genetic engineering can be added to the 5' terminal of the artificial RNA molecule of the present invention in order to stabilize the molecule. For example, triphosphate may be added, or a cap structure may be added.

<3' terminal>

A structure commonly used in genetic engineering can be added to the 3' terminal of the artificial RNA molecule of the present invention in order to stabilize the molecule. For example, a phosphate group may be added.

<Other sequences>

The RNA molecule of the present invention may comprise polynucleotides other than the above-described polynucleotide (a), polynucleotide (b) and polynucleotide (c). Examples of other sequences include, for example, polynucleotides for gene expression and drug delivery, and the like. More specifically, examples of other sequences include, for example, a polynucleotide comprising a promoter sequence of an expression vector, a polynucleotide comprising a transcription termination sequence of an expression vector, a polynucleotide comprising a sequence for avoiding degradation by an RNA nuclease enzyme, a polynucleotide comprising a sequence having affinity for a splicing control factor, and the like.

<Target gene>

The artificial RNA molecule of the present invention targets a pre-mRNA with the polynucleotide (b), thereby regulating splicing of the pre-mRNA, so that a translated and expressed gene (target gene) can be controlled. Examples of such a target gene include, for example, an FAS gene, a dystrophin gene (DMD), a tau (MAPT) gene, a fukutin gene (FKTN), a survival motor neuron (SMN) 2 gene, a myotonin protein kinase (DMPK) gene, an IKBKAP gene, a GFAP gene, a titin gene (TTN), a C1-INH gene (SERPING1 gene), an eye shut homolog (EYS) gene, a huntingtin gene (HTT), and the like. The target gene may be a gene known to form a plurality of isoforms from a single gene by alternative splicing, or may

be a gene for which no isoforms have generally been identified in vivo. Here, examples of the gene that known to form a plurality of isoforms from a single gene by alternative splicing include, for example, an FAS gene and a tau (MAPT) gene. Examples of the gene for which no isoforms have generally been identified in vivo include, for example, a dystrophin gene (DMD) and a fukutin gene (FKTN).

<Expression vector>

The present invention also provides an expression vector comprising a DNA sequence encoding the above-mentioned artificial RNA molecule of the present invention. Such an expression vector is used to express the artificial RNA of the present invention in vivo, which can be utilized to regulate mRNA splicing of a target gene.

Examples of the expression vector include a plasmid, a bacteriophage, a viral vector, a cosmid, and the like.

Examples of the plasmid include PCDNA3, PCDNA5FRT/TO, and the like.

The viral vector is a vector that is based on one or more nucleic acid sequences comprising a viral genome. Examples of the viral vector include an adenovirus vector, an adeno-associated virus vector (AAV), a retrovirus vector, a lentivirus vector, and the like.

In the present invention, when the viral vector is used as an expression vector, a parvovirus vector such as an AAV vector and the like is preferable. The parvovirus is a small virus having a single-stranded DNA genome. The AAV belongs to a parvovirus family.

As an expression promoter possessed by the expression vector, any promoter of an RNA pol III enzyme may be used since the artificial RNA molecule of the present invention is transcribed by the RNA pol III enzyme, specific examples of which include a U6 promoter, an H1 promoter, and the like, and among them, a U6 promoter is preferable.

The expression vector comprising the DNA sequence encoding the artificial RNA molecule of the present invention can be obtained by chemically synthesizing the artificial RNA molecule of the present invention and all of the expression vectors such as an expression promoter, a plasmid, and the like, or by chemically or biochemically synthesizing the artificial RNA molecule of the present invention and the expression promoter and recombining it into an expression vector, such as a virus, a plasmid, and the like.

As a method of recombining an expression vector so as to comprise a DNA sequence encoding the artificial RNA of the present invention, a general method in molecular biology is used. For example, a method using a restriction enzyme and a ligase or a method using Gibson assembly is used.

In a second embodiment, the present invention provides a method of regulating mRNA splicing (also referred to as a regulating method of the present invention), the method comprising the steps of:

(A) preparing an artificial RNA molecule comprising:

(a) a polynucleotide potentially having a secondary structure represented by the formula (I), or a polynucleotide in which 1 to 3 bases are substituted, deleted, or added among 7 bases on the 3' side of the polynucleotide potentially having the secondary structure represented by the formula (I); and
(b) a pre-mRNA targeting polynucleotide comprising a sequence complementary to a target sequence that is a portion of a pe-mRNA; wherein said (a) and (b) are arranged from the 5' side to the 3' side in this order; and

(B) contacting the artificial RNA molecule prepared in the step (A) with the pre-mRNA to form a complementary strand between the artificial RNA molecule and the pre-mRNA, thereby regulating mRNA splicing.

<Step (A)>

The step (A) is a step of preparing an artificial RNA molecule. Examples of the step of preparing the artificial RNA molecule of the present invention include, but not particularly limited to, a method of chemically synthesizing an RNA molecule, a method of synthesizing an RNA molecule by a biochemical technique, and the like. Examples of the method of chemically synthesizing include a phosphoramidite method or an improved method thereof, an H-phosphonate method or an improved method thereof, and the like. Examples of the method of synthesizing by a biochemical technique include a method of synthesizing an RNA molecule using an in vitro transcription system (enzymatically synthesizing method), and the like. Moreover, a commission-manufactured artificial RNA can also be adapted for the present invention.

<Step (B)>

The step (B) is a step of contacting the artificial RNA molecule prepared in the step (A) with the pre-mRNA to form a complementary strand between the artificial RNA molecule and the pre-mRNA, thereby regulating mRNA splicing. As a method of contacting an artificial RNA molecule with a target pre-mRNA, any method commonly used in the art can be

used, such as a method of introducing an artificial RNA molecule into a cell ex vivo, a method of administering an artificial RNA molecule to a living body, and the like.

As the method of introducing an artificial RNA molecule into a cell ex vivo, any known method can be used, such as, for example, a lipofection method, a liposome method, an electroporation method, a calcium phosphate coprecipitation method, a microinjection method, a gene gun method, a reverse transfection method, and the like.

Here, mRNA splicing normally proceeds with a two-stage mechanism. In the first stage, a 5' splicing site is cleaved, and a free 5' exon and a lariat intermediate are generated. In the second stage, this 5' exon is ligated to a 5' exon with release of an intron as a lariat product. These reactions are catalyzed by a spliceosome (see Moore, M. J., P. A. Sharp (1993) Nature, 365:364-368, etc.).

How mRNA splicing is regulated depends on a type of a splicing control factor attracted by the polynucleotide (a), a target sequence targeted by the polynucleotide (b), etc.

Specific examples of the method of regulating mRNA splicing include a method of inducing intron retention, a method of correcting alternative splicing, and the like, but a method of correcting alternative splicing is preferable. Examples of the method of correcting alternative splicing include a method of inducing inclusion of an exon, a method of inducing skipping of the exon (exon skipping), and the like, and a method of inducing exon skipping is preferable.

Regulation of mRNA splicing can be confirmed by RT-PCR or the like. Specifically, it can be confirmed that a total RNA is extracted from cells or tissues into which the artificial RNA molecule of the present invention is introduced, and mRNA is purified by obtaining cDNA by reverse transcription using a reverse transcriptase, and a mature mRNA in which splicing of a target sequence is regulated is obtained by performing PCR using a PCR primer that specifically amplifies the target sequence.

In a third embodiment, the present invention provides a method of producing a mature mRNA (also referred to as a producing method of the present invention), the method comprising the steps of:

(A) preparing an artificial RNA molecule comprising:

(a) a polynucleotide potentially having a secondary structure represented by the formula (I), or a polynucleotide in which 1 to 3 bases are substituted, deleted, or added among 7 bases on the 3' side of the polynucleotide potentially having the secondary structure represented by the formula (I); and
(b) a pre-mRNA targeting polynucleotide comprising a sequence complementary to a target sequence that is a portion of a pre-mRNA; wherein the (a) and the (b) are arranged from the 5' side to the 3' side in this order;

(B') contacting the artificial RNA molecule prepared in the step (A) with the pre-mRNA to form a complementary strand between the artificial RNA molecule and the pre-mRNA; and
(C) regulating mRNA splicing by the step (B') to produce a mature mRNA.

The mature mRNA obtained by the producing method of the present invention can be used for gene therapy by in vivo and ex vivo approaches. As an in vivo approach, for example, an embodiment can be envisioned such that the artificial mRNA molecule of the present invention is administered into a living body and taken into a nucleus of a cell, and a mature mRNA produced in the living body remains there, thereby producing a normal protein. As an ex vivo approach, for example, a usage form can be envisioned such that a cell or tissue in a living body is taken out, the artificial mRNA molecule of the present invention is administered and taken into a nucleus of the cell, and a mature mRNA produced in the cell remains there, thereby returning the cell or tissue, in which a normal protein is produced, back to the patient.

<Step (B')>

The step (B') is a step of contacting the artificial RNA molecule prepared in the step (A) with the pre-mRNA to form a complementary strand between the artificial RNA molecule and the pre-mRNA. In the step (B'), as in the step (B) of the regulating method of the present invention, any method commonly used in the art can be used, such as a method of introducing an artificial RNA molecule into a cell ex vivo, a method of administering an artificial RNA molecule to a living body, and the like.

<Step (C)>

The step (C) is a step of regulating mRNA splicing by the step (B') to produce a mature mRNA. A confirmation that mRNA splicing is regulated by the step (B') to produce a mature mRNA can be performed by RT-PCR or the like, as in the regulating method of the present invention.

The above-mentioned description of the artificial RNA molecule of the present invention is similarly applied to descriptions of the artificial RNA molecule in the regulating method of the present invention and the producing method of

the present invention. Moreover, applicable contents of the descriptions of the regulating method of the present invention and the producing method of the present invention (particularly the descriptions of the above-mentioned step (A)) are similarly applied to any of the embodiments.

[Pharmaceutical composition]

The present invention can also provide a pharmaceutical composition comprising the artificial RNA molecule of the present invention or the mature mRNA obtained by the producing method of the present invention. This pharmaceutical composition can be used for treatment or prevention of diseases that can be treated by regulating mRNA splicing.

As a drug delivery system (DDS) for such a pharmaceutical composition, a method commonly used in this technical field can be used without particular limitation, examples of which include, for example, a method using an expression vector, a method using a lipid nanoparticle (LNP), a method using chemical modification, and the like.

Specific examples of the method using an expression vector include methods using viral vectors, and non-viral vectors such as plasmids, bacteria, and the like. Among them, viral vectors are widely used in gene therapy because they can introduce and deliver a drug with a relatively high efficiency. Furthermore, among viral vectors, AAV vectors are appropriately used from the viewpoint of safety. When the artificial RNA molecule of the present invention is loaded into AAV, multiple copies can be loaded. Therefore, an expression amount of RNA can be increased, so that it is expected that the effects of the present invention are improved.

The method using a lipid nanoparticles (LNP) is a drug delivery method in which an RNA molecule is encapsulated in the LNP. The RNA molecule is packaged into the LNP that protects the RNA molecule from RNase, enters a cell, and is released into a cytoplasm. The use of the LNP allows for an efficient delivery of a chemically modified or unmodified RNA molecule. To prepare the LNP, a PEGylated lipid, cholesterol, a neutral phospholipid, and the like are required.

The method using chemical modification involves chemically modifying a nucleotide that constitute an artificial RNA molecule or a mature mRNA, specific examples of which include a method of adding a peptide to a nucleotide, a method of adding a 2'O-ME modification, a method of adding a phosphorothioate modification, a method of adding a pseudouridine, and the like. Such chemical modifications can protect the RNA molecule from RNase, specifically delivery it to a target tissue or cell, and strongly bond it to a target sequence.

Examples of the diseases that can be treated by regulating mRNA splicing include systemic lupus erythematosus (SLE), autoimmune lymphoproliferative syndrome (ALPS), Duchenne muscular dystrophy (DMD), frontotemporal dementia (FTDP-17), Fukuyama muscular dystrophy, myotonic dystrophy, amyotrophic lateral sclerosis (ALS), spinal muscular atrophy (SMA), spinal-bulbar muscular atrophy (SBMA), familial dysautonomia (FD), Alexander disease, Angelman syndrome (AS), idiopathic dilated cardiomyopathy (DCM), Parkinson's disease, hereditary angioedema (HAE), retinitis pigmentosa, Huntington's disease, chronic kidney disease, nonalcoholic steatohepatitis (NASH), thalassemia, and various cancers, etc..

EXAMPLES

The present invention will be described in more detail below based on specific Experimental examples and Examples, though the present invention is not limited to these Experimental examples and Examples.

Experimental example 1: Creation of knockout mouse

First, a 4.5SH knockout mouse was created. The knockout mouse was created according to a general method for a large-scale genomic deletion/insertion method using a CRISPR/Cas9 system (see Luqing Zhang et al., (2015) PLoS One, Mar 24;10(3), etc.). Specifically, a gene cassette comprising a drug resistance gene having a sequence at both ends complementary to that at both ends of a mouse 4.5SH gene cluster was designed and isolated, and the isolated gene was introduced into a mouse ES cell by electroporation. To increase an efficiency of homologous recombination, CRISPR/-Cas9, which cleaves terminal portions of the complementary sequence, was simultaneously introduced.

A chimeric mouse was produced from an established ES cell and mated with a wild-type mouse to obtain a recombinant heterozygous mouse. Heterozygous mice were mated with each other to obtain a blastocyst of the 4.5SH knockout mouse. A 4.5SH knockout ES cell was obtained from the blastocyst. RNA was extracted from the ES cell of the 4.5SH knockout mouse, and RT-PCR was performed using a primer that specifically amplifies 4.5SH with cDNA synthesized from RNA being as a template. As a result, 4.5SH was significantly reduced in the 4.5SH knockout mouse.

Experimental example 2: RNA-Seq analysis

A total RNA was prepared from an established ES cell of a 4.5SH knockout mouse and an established ES cell of a

wild-type mouse using Trizol. The prepared total RNA was subjected to RNA-Seq analysis. For sequencing, HiSeq4000 manufactured by Illumina, Inc. was used. A change in alternative splicing was compared among three groups for each of the ES cell of the 4.5SH knockout mouse and the cell of the wild-type mouse using rMATS 4.1.1 as an analysis software.

Table 1 shows results of comparisons at FDR (False Discovery Rate) of less than 0.05 for changes of five types of statistically significant alternative splicing in the ES cell of the 4.5SH knockout mouse and the cell of the wild-type mouse. Here, the five types of alternative splicing refer to a cassette exon type (SE), an alternative 5' splice site type (A5SS), an alternative 3' splice site type (A3SS), a mutually exclusive exon type (MXE), and an intron retention type (IR).

Table 1

| Type | Total event | Significant event | Significant exclusion event | Significant inclusion event |
|---|---|---|---|---|
| SE | 12058 | 508 | 215 | 293 |
| A5SS | 6537 | 219 | 61 | 158 |
| A3SS | 9791 | 274 | 87 | 187 |
| MXE | 784 | 85 | 33 | 52 |
| IR | 8407 | 1349 | 219 | 1130 |

As a result of the RNA-Seq analysis, it was found that there were 293 exons specifically appearing in the ES cell of the 4.5SH knockout mouse.

Next, analyzing features of the exons specifically appearing in the ES cell of the 4.5SH knockout mouse from the results of RNA-Seq analysis, it was found that many of the exons specifically appearing in the ES cell of the 4.5SH knockout mouse matched a sequence of asSINEB1. Here, asSINEB1 is a complementary strand (as) of SINEB1. Therefore, a hypothesis was set up which, in Myomorpha, 4.5SH suppresses asSINEB1 present within an intron from exonizing, which was demonstrated through the following Experimental examples.

Experimental example 3: Confirmation of exon skipping in mouse cell

RNA was extracted from an ES cell of each of male and female 4.5SH knockout mice and wild-type mice, and RT-PCR was performed using primers (SEQ ID NO: 3 and NO: 4, SEQ ID NO: 5 and NO: 6, and SEQ ID NO: 7 and NO: 8) that specifically amplify three genes having asSINEB1 (Smchd1 exons 21-22, Slc25a40 exons 8-9, and Smg6 exons 8-9), respectively, with cDNA synthesized from RNA being as a template. In introns, asSINEB1s are thereby present between an exon 24 and an exon 25 of Smchd1 (NM_028887.3), between an exon 8 and an exon 9 of SIc25a40 (NM_178766.5), and between an exon 8 and an exon 9 of Smg6 (NM_001002764.1), and therefore, if these asSINEB1s are exonized to become de novo exons by knocking out of 4.5SH, this case will be one to prove the demonstration of the above-described hypothesis.

As a result, for Smcdh1, Slc25a40, and Smg6, exon skipping of a de novo exon was detected in the cell of the wild-type mouse, whereas no exon skipping of a de novo exon was detected in the ES cell of the 4.5SH knockout mouse (FIG. 2). Thus, the de novo exon was skipping in the presence of 4.5SH, but not in the presence of 4.5SH knockout, confirming appearance of the de novo exon. Hereinafter, in the present specification and drawings, these de novo exons are also referred to as Smcdh1 Ex24.5, SIc25a40 Ex8.5, and Smg6 Ex8.5, respectively, or as Smcdh1 exon 24.5, SIc25a40 exon 8.5, and Smg6 exon 8.5, respectively.

Experimental example 4: Confirmation of exon skipping in human cell overexpression system

For the purpose of further demonstrating the above-described hypothesis by confirming activity of 4.5SH using a human cell that does not naturally have 4.5SH, exon skipping was confirmed in a human cell overexpression system.

A plasmid carrying a U6 promoter and a mouse 4.5SH sequence (FIG. 3) was prepared, and the plasmid (pUC57) was introduced into a human cell (HEK293 cell) by a liposome transfection method. Expression of 4.5SH RNA was confirmed by Northern blotting, and a human 4.5SH-expressing cell was obtained (FIG. 4).

Minigenes in which a DNA sequence (SEQ ID NO: 9) comprising a wild-type Slc25a40Ex8.5 and 100 bases upstream and downstream thereof and a DNA sequence (SEQ ID NO: 10) comprising a wild-type Smg6Ex8.5 and 100 bases upstream and downstream thereof were inserted into an SacII sequence in an intron of a plasmid derived from an adenovirus late gene (Ad2), respectively, were inserted into a plasmid (pcDNA3 BamHI/XbaI), respectively, and the obtained vectors (Ad2/pcDNA3 BamHI/XbaI, the common sequence of the vectors is SEQ ID NO: 28) were introduced into a human 4.5SH-expressing cell and cultured at 37°C for 24 hours, after which the cell was recovered and RNA was extracted. RT-PCR was performed using primers (SEQ ID NO: 26 and SEQ ID NO: 27) that both specifically amplify two

minigenes with cDNA synthesized from RNA being as a template. As a result, exon skipping was detected for the minigenes of Slc25a40 exon 8.5 and Smg6 exon 8.5 (FIG. 5). Besides, the left column in each of FIGS. 5 and 6 shows results for a control in which the minigenes were introduced into a human cell (HEK293 cell) prior to 4.5SH introduction, instead of into the human 4.5SH-expressing cell.

10 μL of reaction product with the above-described PCR was electrophoresed in a polyacrylamide gel, and an exon skipping efficiency was calculated from each band on the obtained electrophoretic view. An exon skipping efficiency was calculated from a polynucleotide amount (X) and a nucleotide length ($L_x$) of a band skipped by an exon and a polynucleotide amount (Y) and a nucleotide length ($L_y$) of a band skipped by the exon, by the following equation (FIG. 6):

$$\text{Skipping efficiency (\%)} = \{X/L_x \div (X/L_x + Y/L_y)\} \times 100$$

From the descriptions above, it was found that, even in a human cell that does not naturally have 4.5SH, a de novo exon in an intron appears in the absence of 4.5SH, and a de novo exon is skipped in the presence of 4.5SH.

From Experimental examples 3 and 4, it was found that 4.5SH suppresses asSINEB1 present in an intron from exonizing, and that the suppression of exonization is due to exon skipping of a de novo exon.

Experimental example 5: Examination of base pairing by co-expression using mutant type

For the purpose of investigating base pairing between 4.5SH and asSINEB1, mutant types of 4.5SH and asSINEB1 were obtained, respectively, and co-expressed in a cell to detect presence or absence of exon skipping.

Mutation was introduced into a plasmid expressing a natural (wild-type) 4.5SH obtained in the same manner as in Experimental example 4 to obtain a plasmid expressing a mutant type. That is, a plasmid was prepared in which a partial G in an asSINEB1 recognition region of a wild-type 4.5SH sequence was modified to C and a sequence of a mouse 4.5SH was modified so as not to base-pair with a de novo exon (exon 8.5) between Slc25a40 exons 8 and 9 (FIG. 7). The obtained plasmid was transformed into E. coli. The transformed E. coli was plated on an LB (containing ampicillin) plate to allow a colony to be formed at 37°C, and screening was performed. A DNA sequence of the plasmid held by ampicillin-resistant E. coli was determined to obtain a mutant-type 4.5SH-expressing plasmid (FIG. 7).

Mutation was introduced into a plasmid expressing a minigene comprising a natural (wild-type) Slc25a40 exon 8.5 obtained in the same manner as in Experimental example 4 to obtain a plasmid expressing a mutant type. That is, a fragment of a DNA sequence (SEQ ID NO: 11) designed so as not to base-pair with a wild-type 4.5SH, in which a partial G in a wild-type Slca40 exon 8.5 sequence was modified to C, was synthesized, and mutation was inserted into the plasmid by Gibson assembly. The obtained plasmid was transformed into E. coli. The transformed E. coli was plated on an LB (containing ampicillin) plate to allow a colony to be formed at 37°C, and screening was performed. A DNA sequence of the plasmid held by ampicillin-resistant E. coli was determined to obtain a plasmid expressing a minigene comprising a mutant-type Slc25a40 exon 8.5 (FIG. 7).

Each mutant-type plasmid and each wild-type plasmid as described above were co-transfected into a human cell (HEK293 cell) by a lipofection method. After culturing for 24 hours at 37°C, the cell was recovered and RNA was extracted. RT-PCR was performed using primers (SEQ ID NO: 26 and SEQ ID NO: 27) that specifically amplify both minigenes used also in Experimental example 4 with cDNA synthesized from RNA being as a template. 10 μL of reaction product with the PCR was electrophoresed in a polyacrylamide gel, and an exon skipping efficiency was calculated by the above-mentioned equation. The results are shown in FIGS. 8 and 9. Besides, in FIGS. 8 and 9, the leftmost lanes and bar graphs show a sample into which a plasmid before 4.5SH was introduced (so-called an empty vector) and a minigene-expressing plasmid comprising a wild-type Slc25a40 exon 8.5 were co-transfected.

Exon skipping was detected in a cell where a wild-type 4.5SH and a minigene comprising a wild-type Slc25a40 exon 8.5 were co-expressed (the second lane from the left in FIG. 8). Exon skipping was not detected or a skipping efficiency was lower compared with a case where wild types were co-expressed with each other, in a cell where a mutant-type 4.5SH and a minigene comprising a wild-type Slc25a40 exon 8.5 were co-expressed and in a cell where a wild-type 4.5SH and a minigene comprising a mutant-type Slc25a40 exon 8.5 were co-expressed (the third and fourth lanes from the left in FIG. 8). Exon skipping was detected and a skipping efficiency was equivalent to a case where wild types were co-expressed with each other, in a cell where a mutant-type 4.5SH and a minigene comprising a mutant-type Slc25a40 exon 8.5 were co-expressed (the fifth lane from the left in FIG. 8; the fifth lane from the left for the skipping efficiency in FIG. 9). As described above, it can be found that exon skipping with 4.5SH requires base pairing between 4.5SH and asSINEB1.

Experimental example 6: Analysis of polynucleotide (a)

For the purpose of confirming that the polynucleotide (a) is important in 4.5SH, a B1-modified 4.5SH was prepared in which the sequence of the polynucleotide (a) part of 4.5SH (SEQ ID NO: 2) was substituted with a sequence of

a corresponding part of SINEB1 that is a homologous sequence of 4.5SH (an RNA sequence corresponding to SEQ ID NO: 28), and an expression amount and an exon skipping efficiency in a cell were examined.

<Acquisition of B1-modified 4.5SH>

Mutation was introduced into a plasmid expressing a wild-type 4.5SH obtained in the same manner as in Experimental example 4 to obtain a plasmid expressing a B1-modified 4.5SH. Specifically, a sequence of the polynucleotide (a) part of the wild-type 4.5SH (a DNA sequence corresponding to SEQ ID NO: 2) was modified to a DNA sequence of a corresponding part of SINEB1 (SEQ ID NO: 29) to prepare a plasmid (pUC57) carrying a U6 promoter on the 5' side of the DNA sequence. The obtained plasmid was transformed into E. coli. The transformed E. coli was plated on an LB (containing ampicillin) plate to allow a colony to be formed at 37°C, and screening was performed. A DNA sequence of the plasmid held by ampicillin-resistant E. coli was determined to obtain a B1-modified 4.5SH-expressing plasmid shown in SEQ ID NO: 30.

The B1-modified 4.5SH-expressing plasmid obtained above was introduced into a human cell (HEK293 cell) by a liposome transfection method to obtain a B1-modified 4.5SH-expressing cell. Expression of the B1-modified 4.5SH RNA was confirmed by Northern blotting (the middle level in FIG. 10, the rightmost lane: SL1-B1-4.5SH).

<Comparison in skipping efficiency between B1-modified 4.5SH and wild-type 4.5SH>

Minigenes comprising a wild-type Slc25a40 exon 8.5 and 100 bases upstream and downstream thereof, obtained in the same manner as in Experimental example 4, were introduced into a wild-type 4.5SH-expressing cell (obtained in the same manner as for the human 4.5SH-expressing cell in Experimental example 4) and the B1-modified 4.5SH-expressing cell obtained above, which were cultured at 37°C for 24 hours, after which the cells were recovered and RNAs were extracted. RT-PCR was performed using primers (SEQ ID NO: 26 and SEQ ID NO: 27) that specifically amplify the minigene comprising the wild-type Slc25a40Ex8.5 used also in Experimental example 4 with cDNA synthesized from RNA being as a template. As a result, for the Slc25a40 exon 8.5, an exon skipping efficiency was significantly reduced (the lower level in FIG. 10) in the B1-modified 4.5SH-expressing cell (the rightmost lane in FIG. 10) compared to the wild-type 4.5SH-expressing cell (the center lane in FIG. 10). Besides, the leftmost column in FIG. 10 shows results for a control in which, instead of using the human 4.5SH-expressing cell or the B1-modified 4.5SH-expressing cell, a plasmid before introduction of a mouse 4.5SH in Experimental example 4 (so-called an empty vector) was introduced into a human cell (HEK293 cell).

Example 1: FAS gene-modified 4.5SH

For the purpose of confirming that exon skipping of asSINEB1 by 4.5SH can be applied to any exon skipping, a human FAS gene-modified 4.5SH was prepared and exon skipping was examined.

<Acquisition of FAS gene-modified 4.5SH>

Mutation was introduced into a plasmid expressing a wild-type 4.5SH to obtain a plasmid expressing a mutant type. Specifically, DNA fragments were synthesized in which a sequence of an asSINEB1 recognition region of the wild-type 4.5SH sequence was modified to a DNA sequence complementary to an FAS gene exon 6 shown in SEQ ID NO: 12, and inserted into a plasmid (pUC57) by Gibson assembly. The obtained plasmid was transformed into E. coli. The transformed E. coli was plated on an LB (containing ampicillin) plate to allow a colony to be formed at 37°C, and screening was performed. A DNA sequence of the plasmid held by ampicillin-resistant E. coli was determined to obtain a human FAS gene-modified 4.5SH-expressing plasmid shown in SEQ ID NO: 13.

<Preparation of minigene for detecting exon skipping>

A DNA fragment comprising FAS genes exon 5, intron 5, exon 6, intron 6, and exon 7 shown in SEQ ID NO: 14 was amplified by PCR, the PCR product was purified, and mutation was inserted into a plasmid (pcDNA3 bamHI/xhol) by Gibson assembly. The obtained plasmid was transformed into E. coli. The transformed E. coli was plated on an LB (containing ampicillin) plate to allow a colony to be formed at 37°C, and screening was performed. A DNA sequence of the plasmid held by ampicillin-resistant E. coli was determined to obtain a minigene for detecting exon skipping of an FAS gene.

<Detection of exon skipping of FAS gene>

The FAS gene-modified 4.5SH-expressing plasmid and the minigene for detecting exon skipping of the FAS gene as obtained above were co-transfected into a human cell (HEK293 cell) by a lipofection method. After culturing for 24 hours at 37°C, the cell was recovered and RNA was extracted. RT-PCR was performed using a reverse primer and a forward primer that bind to pcDNA3 shown in SEQ ID NO: 15 and SEQ ID NO: 16 with cDNA synthesized from RNA being as a template. The FAS gene present in the cell is not amplified by using such a primer set. RT-PCR conditions are shown in Table 2.

Table 2

| | Temperature | Time | Cycle number |
|---|---|---|---|
| Thermal denaturation | 95°C | 30 seconds | 23 cycles |
| Annealing | 58°C | 30 seconds | |
| Extension | 72°C | 1 minute | |

10 μL of reaction product with the PCR was electrophoresed in a polyacrylamide gel, and skipping was analyzed. The electrophoretic view is shown in the right lane of FIG. 11. Exon skipping efficiencies are shown in FIG. 13.

In a cell in which the FAS gene-modified 4.5SH-expressing plasmid and the minigene for detecting exon skipping of the FAS gene were co-expressed, exon skipping of the FAS gene exon 6 was confirmed (the right lane in FIG. 11). Moreover, an exon skipping efficiency of the FAS gene exon 6 by the FAS gene-modified 4.5SH was 70% (the rightmost bar inf FIG. 13).

The left lane of the electrophoresis view in FIG. 11 is an electrophoresis view where RNA, in which a wild-type 4.5SH-expressing plasmid and a minigene for detecting exon skipping of an FAS gene were co-expressed into a human cell (HEK293 cell) and extracted, was subjected to RT-PCR. An exon skipping efficiency was significantly lower than that of the FAS gene-modified 4.5SH-expressing plasmid.

<Confirmation of exon skipping by FAS gene-modified 4.5SH>

The FAS gene-modified 4.5SH-expressing plasmid obtained above and a plasmid expressing a minigene comprising a wild-type SIc25a40 exon 8.5 obtained in the same manner as in Experimental example 4 were co-transfected into a human cell (HEK293 cell) by a lipofection method. After culturing for 24 hours at 37°C, the cell was recovered and RNA was extracted. RT-PCR was performed using primers (SEQ ID NO: 26 and SEQ ID NO: 27) that specifically amplify minigenes used also in Experimental example 4 with cDNA synthesized from RNA being as a template. 10 μL of reaction product with the PCR was electrophoresed in a polyacrylamide gel, and skipping was analyzed. An image of the electrophoretic view is shown in the right lane of FIG. 12.

The left lane of the electrophoresis view in FIG. 12 is an electrophoresis view where RNA, in which a wild-type 4.5SH-expressing plasmid and a plasmid expressing a minigene comprising a wild-type Slc25a40 exon 8.5 were co-expressed into a human cell (HEK293 cell) and extracted, was subjected to RT-PCR. By comparing the results of co-expression of the wild-type 4.5SH-expressing plasmid and the wild-type SIc25a40 exon 8.5 (the left lane in FIG. 12 and the leftmost bar in FIG. 13) with the results of co-expression of the FAS gene-modified 4.5SH and the minigene for detecting exon skipping of the FAS gene (the right lane in FIG. 11 and the rightmost bar in FIG. 13), it can be found that the FAS gene-modified 4.5SH has a skipping activity of the FAS gene exon 6 equivalent to a skipping activity of the wild-type 4.5SH against asSINEB1 (FIGS. 11, 12, and 13). Besides, since it is known that the FAS gene has an isoform that contains exon 6 and an isoform that does not contain exon 6 in a living body, the results of co-expression of the wild-type 4.5SH-expressing plasmid and the minigene for detecting exon skipping of the FAS gene (the right lane in FIG. 11 and the third bar from the right in FIG. 13) are considered to indicate an isoform amount ratio in the living body.

Example 2: Human DMD gene-modified 4.5SH

For the purpose of confirming that exon skipping of asSINEB1 by 4.5SH can be applied to any exon skipping, a human dystrophin (DMD) gene-modified 4.5SH was prepared and exon skipping was examined. Since exon 53 of DMD is 212 bases long, which is longer than the FAS gene exon 6 (63 bases), a target sequence was first divided into four locations (#1, #2, #3, #4) (see FIG. 14) to prepare four DMD gene-modified 4.5SHs (DMD gene-modified 4.5SHs #1 to #4) with different base sequences of the polynucleotide (b).

<Acquisition of four DMD gene-modified 4.5SHs>

Mutation was introduced into a plasmid expressing a wild-type 4.5SH to obtain four types of plasmids expressing a mutant type. Specifically, DNA sequences were designed in which the sequence of the asSINEB1 recognition region of the wild-type 4.5SH sequence was modified to a DNA sequence complementary to a DMD gene exon 53 shown in SEQ ID NO: 17, SEQ ID NO: 18, SEQ ID NO: 19, and SEQ ID NO: 20.

DNA fragments in which the DNA sequences were modified to DMD sequences complementary to the DMD gene exon 53 were synthesized, respectively, and mutation was inserted into a plasmid (pcDNA3 bamHI/xhol) by Gibson assembly. The obtained plasmid was transformed into E. coli. The transformed E. coli was plated on an LB (containing ampicillin) plate to allow a colony to be formed at 37°C, and screening was performed. DNA sequences of plasmids held by ampicillin-resistant E. coli were determined to obtain four types of DMD gene-modified 4.5SH-expressing plasmids shown in SEQ ID NO: 21, SEQ ID NO: 22, SEQ ID NO: 23, and SEQ ID NO: 24.

<Preparation of minigene for detecting exon skipping>

A DNA sequence comprising a DMD gene exon 53 and 100 bases upstream and downstream thereof as shown in SEQ ID NO: 25 was inserted into an SacII sequence in an intron of a plasmid derived from an adenovirus late gene (Ad2) to obtain a minigene for detecting exon skipping of a DMD gene.

<Detection of exon skipping of DMD gene>

The above-described DMD gene-modified 4.5SH-expressing plasmid and a vector in which the above-described minigene for detecting exon skipping of the DMD gene was inserted into a plasmid (Ad2/pcDNA3 BamHI/Xbal, the common sequence of the vectors is SEQ ID NO: 28) were co-transfected into a human cell (HEK293 cell) by a lipofection method. Each of the DMD gene-modified 4.5SH-expressing plasmids (plasmids expressing DMD gene-modified 4.5SHs #1 to #4, respectively) and a mixture of the all four types were co-transfected with a minigene insertion plasmid for detecting exon skipping of the DMD gene. After culturing for 24 hours at 37°C, the cell was recovered and RNA was extracted. RT-PCR was performed using primers (SEQ ID NO: 26 and SEQ ID NO: 27) that specifically amplify each minigene used also in Experimental example 4 with cDNA synthesized from RNA being as a template. The RT-PCR conditions are shown in Table 3.

Table 3

| | Temperature | Time | Cycle number |
|---|---|---|---|
| Thermal denaturation | 95°C | 30 seconds | 40 cycles |
| Annealing | 58°C | 30 seconds | |
| Extension | 72°C | 1 minute | |

10 μL of reaction product with the PCR was electrophoresed in a polyacrylamide gel, and skipping was analyzed. An image of the electrophoretic view is shown in FIG. 15.

In the DMD gene exon 53, skipping was detected, although the skipping efficiency was not high. In particular, in the DMD gene-modified 4.5SH #2 (simply represented as "2" in FIG. 15) and a sample in which all of the four DMD gene-modified 4.5SHs #1 to #4 were mixed ("Mix" in FIG. 15), bands after exon skipping could be confirmed (the third lane from the left and the sixth lane from the left in FIG. 15).

Example 2-2: Optimization of human DMD gene-modified 4.5SH

<Design of polynucleotide (b) sequence>

Furthermore, in order to prepare a DMD gene-modified 4.5SH having a high skipping efficiency, a target sequence of DMD exon 53 was optimized. It was found that the sequence of #4 (SEQ ID NO: 20), which is a part of DMD exon 53, comprised two transcription termination signal sequences (specifically, TTTT) recognized by the RNA pol III enzyme, terminating the transcription. Therefore, the relevant site was converted to TATT to be designated as #5 (SEQ ID NO: 31, FIG. 17). This DNA sequence was used to prepare a DMD gene-modified 4.5SH #5 in the same manner as in Example 2. FIG. 17 shows a schematic view of a site complementary to an exon of DMD exon 53 of each sequence.

<Acquisition of DMD gene-modified 4.5SH>

DNA fragments were synthesized in which the sequences of the asSINEB1 recognition region of the wild-type 4.5SH sequence were modified to DNA sequences shown in SEQ ID NO: 17, SEQ ID NO: 18, SEQ ID NO: 19, and SEQ ID NO: 31 (each of which is a DNA sequence complementary to a part of DMD gene exon 53) to obtain four types of DMD gene-modified 4.5SH #1, #2, #3, and #5-expressing plasmids shown in SEQ ID NO: 21, SEQ ID NO: 22, SEQ ID NO: 23, and SEQ ID NO: 32.

The DMD gene-modified 4.5SH-expressing plasmid and the minigene for detecting exon skipping of the DMD gene prepared in the same manner as in Example 2 were co-transfected into a human cell (HEK293 cell) by a lipofection method. Here, all possible two types of combination of DMD gene-modified 4.5SH-expressing plasmids were co-introduced with the minigene for detecting exon skipping of the DMD gene. After culturing for 24 hours at 37°C, the cell was recovered and RNA was extracted. RT-PCR was performed using primers (SEQ ID NO: 26 and SEQ ID NO: 27) that specifically amplify minigenes used also in Experimental example 4 with cDNA synthesized from RNA being as a template. The RT-PCR conditions are shown in Table 4.

Table 4

| | Temperature | Time | Cycle number |
|---|---|---|---|
| Thermal denaturation | 95°C | 30 seconds | 35 cycles |
| Annealing | 58°C | 30 seconds | |
| Extension | 72°C | 1 minute | |

1 μL of reaction product with the PCR was analyzed using BioAnalyzer (manufactured by Agilent Technologies Japan, Ltd.), and an exon skipping efficiency was calculated. As a result, in all combinations, skipping was detected although the skipping efficiency was not high in some cases. The combination of the DMD gene-modified 4.5SH #2 and the DMD gene-modified 4.5SH #5 showed the highest exon skipping efficiency (the rightmost lane in FIG. 16). Besides, in FIG. 16, the leftmost lane shows results of a sample into which the plasmid prior to introduction of a mouse 4.5SH in Experimental example 4 (so-called the empty vector) was co-transfected with the minigene for detecting exon skipping of the DMD gene. In addition, the second lane from the left shows results of a sample into which the vector expressing the wild-type 4.5SH RNA (the same as the plasmid (pUC57) prepared in obtaining the human 4.5SH-expressing cell in Experimental example 4) was co-transfected with the minigene for detecting exon skipping of the DMD gene.

<Optimization of polynucleotide (b) sequence>

Here, the DNA sequence (SEQ ID NO: 18) corresponding to the polynucleotide (b) of the present invention in the DMD gene-modified 4.5SH #2 was located in the vicinity of a sequence of an existing antisense nucleic acid drug, viltolarsen (see FIG. 17). Therefore, the sequence of the polynucleotide (b) of the DMD gene-modified 4.5SH #2 was redesigned to include all of sequences of viltolarsen, a DMD gene-modified 4.5SH #6 shown in SEQ ID NO: 33 was designed, and a DNA fragment expressing a sequence of a DMD gene-modified 4.5SH #6 was synthesized, to obtain a DMD gene-modified 4.5SH-expressing plasmid expressing the DMD gene-modified 4.5SH #6 shown in SEQ ID NO: 34. Furthermore, a DMD gene-modified 4.5SH #7 was designed which comprises a sequence (SEQ ID NO: 35) in which the sequences of the polynucleotide (b) of the DMD gene-modified 4.5SH #6 and of the polynucleotide (b) of the DMD gene-modified 4.5SH #5 were linked to each other, and a DNA fragment expressing the DMD gene-modified 4.5SH #7 was synthesized, to obtain a DMD gene-modified 4.5SH-expressing plasmid expressing #7 shown in SEQ ID NO: 36.

<B1-type human DMD gene-modified 4.5SH (Comparative example)>

In addition, Comparative example was designed which is a B1-modified sequence in which the polynucleotide (a) of 4.5SH of the DMD gene-modified 4.5SH #7 was substituted with a sequence corresponding to the polynucleotide (a) part of SINEB1, which is a homologous sequence of 4.5SH, and DNA fragments expressing the sequence in Comparative example were synthesized, to obtain a DMD gene-modified B1-modified 4.5SH-expressing plasmid expressing Comparative example shown in SEQ ID NO: 37.

The DMD gene-modified B1-modified 4.5SH-expressing plasmid as obtained above and the minigene for detecting exon skipping of the DMD gene as obtained in Example 2 were co-transfected into a human cell (HEK293 cell) by a lipofection method. After culturing for 24 hours at 37°C, the cell was recovered and RNA was extracted. RT-PCR was performed using primers (SEQ ID NO: 26 and SEQ ID NO: 27) that specifically amplify the minigene for detecting exon skipping of the DMD gene with cDNA synthesized from RNA being as a template. The RT-PCR conditions are shown in

Table 5.

Table 5

| | Temperature | Time | Cycle number |
|---|---|---|---|
| Thermal denaturation | 95°C | 30 seconds | 23 cycles |
| Annealing | 58°C | 30 seconds | |
| Extension | 72°C | 1 minute | |

As a result, in the combination of the DMD gene-modified 4.5SH #6 and the DMD gene-modified 4.5SH #5, a skipping efficiency of DMD exon 53 was 16.49% (the fourth lane from the left in FIG. 18). Then, a skipping efficiency of DMD exon 53 of the DMD gene-modified 4.5SH #7 was 46.92% (the fifth lane from the left in FIG. 18).

Moreover, a skipping efficiency of DMD exon 53 of the sequence in Comparative example was 5.06%, which was significantly reduced (the sixth lane from the left in FIG. 18). Besides, in FIG. 18, the leftmost lane shows results of a sample into which a plasmid prior to 4.5SH introduction (so-called an empty vector) was co-transfected with the minigene for detecting exon skipping of the DMD gene, and the second lane from the left shows results of a sample into which the vector expressing the wild-type 4.5SH RNA (the same as the plasmid (pUC57) prepared in obtaining the human 4.5SH-expressing cell in Experimental example 4) was co-transfected with the minigene for detecting exon skipping of the DMD gene. Since exon skipping does not occur in a living body for the DMD gene, a skipping efficiency was 0% for the control where the empty vector was introduced.

Example 3: MAPT gene-modified 4.5SH

For the purpose of confirming that exon skipping of asSINEB1 by 4.5SH can be applied to any exon skipping, a tau (MAPT) gene-modified 4.5SH was prepared and exon skipping was examined.

<Acquisition of MAPT gene-modified 4.5SH>

DNA fragments were synthesized in which a sequence of an asSINEB1 recognition region of the wild-type 4.5SH sequence was modified to a DNA sequence complementary to an MAPT gene exon 10 shown in SEQ ID NO: 38, and mutation was inserted into a plasmid (pcDNA3 bamHI/xhoI) by Gibson assembly. The obtained plasmid was transformed into E. coli. The transformed E. coli was plated on an LB (containing ampicillin) plate to allow a colony to be formed at 37°C, and screening was performed. A DNA sequence of the plasmid held by ampicillin-resistant E. coli was determined to obtain a human MAPT gene-modified 4.5SH-expressing plasmid shown in SEQ ID NO: 39. A reporter carrying a U6 promoter and an MAPT gene-modified 4.5SH sequence was prepared, and an expression vector (pUC57) carrying the reporter was introduced into a human cell (HEK293 cell) by a liposome transfection method. Expression of the MAPT gene-modified 4.5SH RNA was confirmed by Northern blotting (the middle level in FIG. 20, the rightmost lane), and an MAPT gene-modified 4.5SH-expressing cell was obtained.

<Preparation of minigene for detecting exon skipping>

A DNA fragment comprising MAPT genes exon 9, exon 10, and exon 11, etc. (see Qingming Yu et al, (2004) J Neurochem, Jul; 90 (1): 164-72) shown in SEQ ID NO: 40 was amplified by PCR, the PCR product was purified, and mutation was inserted into a plasmid (pcDNA3 bamHI/xhoI) by Gibson assembly. The obtained plasmid was transformed into E. coli. The transformed E. coli was plated on an LB (containing ampicillin) plate to allow a colony to be formed at 37°C, and screening was performed. A DNA sequence of the plasmid held by ampicillin-resistant E. coli was determined to obtain a minigene for detecting exon skipping of an MAPT gene.

<Detection of exon skipping of MAPT gene>

The MAPT gene-modified 4.5SH-expressing plasmid and the minigene for detecting exon skipping of the MAPT gene as obtained above were co-transfected into a human cell (HEK293 cells) by a lipofection method. After culturing for 24 hours at 37°C, the cell was recovered and RNA was extracted. RT-PCR was performed using a reverse primer that bind to pcDNA3 shown in SEQ ID NO: 41 and SEQ ID NO: 42 and a forward primer that binds to an MAPT gene exon 11 with cDNA synthesized from RNA being as a template. The MAPT gene present in the cell is not amplified by using such a primer set. The RT-PCR conditions are shown in Table 6.

Table 6

| | Temperature | Time | Cycle number |
|---|---|---|---|
| Thermal denaturation | 95°C | 30 seconds | 23 cycles |
| Annealing | 58°C | 30 seconds | |
| Extension | 72°C | 1 minute | |

1 μL of reaction product with the PCR was analyzed using BioAnalyzer (manufactured by Agilent Technologies Japan, Ltd.), and results of calculating exon skipping efficiencies are shown in FIG. 19.

In a cell in which the MAPT gene-modified 4.5SH-expressing plasmid and the minigene for detecting exon skipping of the MAPT gene were co-expressed, exon skipping of the MAPT gene exon 10 was confirmed (the rightmost lane of the electrophoretic view in the upper level in FIG. 19). Moreover, an exon skipping efficiency of the MAPT gene exon 10 by the MAPT gene-modified 4.5SH was 88.78% (the rightmost in the lower level for 4.5SH-asMAPT in FIG. 19).

The lane in the middle (the second from the left) of the electrophoresis view in FIG. 19 is an electrophoresis view in which the FAS-modified 4.5SH-expressing plasmid obtained in Example 1 was co-expressed with the minigene for detecting exon skipping of the MAPT gene in a human cell, and the extracted RNA was subjected to RT-PCR. An exon skipping efficiency was 64.99%, which was significantly lower than the skipping efficiency when the above-described MAPT gene-modified 4.5SH-expressing plasmid was expressed (4.5SH-asFAS in FIG. 19). Besides, in FIG. 19, the leftmost control shows results of a sample into which a plasmid prior to mutation introduction (so-called an empty vector) was co-transfected with the minigene for detecting exon skipping of the MAPT gene. Since it is known that the MAPT gene has an isoform that contains exon 10 and an isoform that does not contain exon 10 in a living body, a skipping efficiency of the control in which the empty vector was introduced is considered to indicate an isoform amount ratio in the living body.

INDUSTRIAL APPLICABILITY

According to the present invention, a technique in which an artificial RNA molecule comprising a sequence of a non-coding RNA molecule specific to Rodentia and Myomorpha animals controls gene expression through regulation of mRNA splicing, so that it can be utilized to treat diseases that can be treated by regulating mRNA splicing.

**Claims**

**1.** An artificial RNA molecule comprising:

(a) a polynucleotide potentially having a secondary structure represented by the following formula (I), or a polynucleotide in which 1 to 3 bases are substituted, deleted, or added among 7 bases on the 3' side of the polynucleotide potentially having the secondary structure represented by the following formula (I):

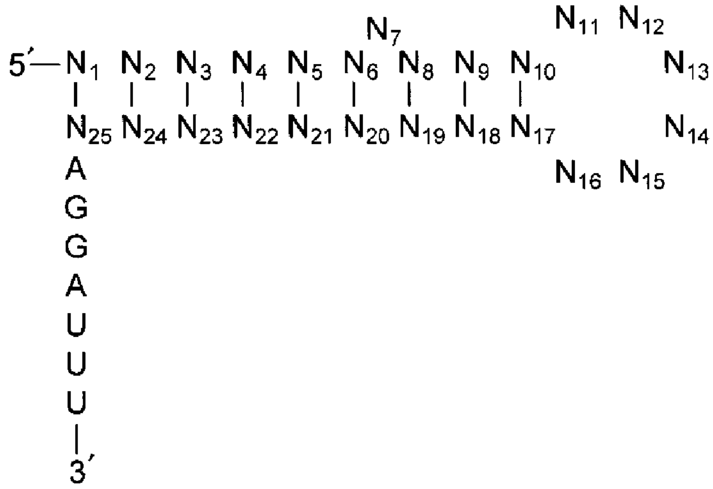


Formula ( I )

(wherein, $N_1$ to $N_{25}$ each independently represents A, C, G, or U, and $N_1$ and $N_2$s, $N_2$ and $N_{24}$, $N_3$ and $N_{23}$, $N_4$ and $N_{22}$, $N_5$ and $N_{21}$, $N_6$ and $N_{20}$, $N_8$ and $N_{19}$, $N_9$ and $N_{18}$, and $N_{10}$ and $N_{17}$, respectively, form a base pair); and

(b) a pre-mRNA targeting polynucleotide comprising a sequence complementary to a target sequence that is a portion of a pre-mRNA;

wherein the (a) and the (b) are arranged from the 5' side to the 3' side in this order.

**2.** The artificial RNA molecule of claim 1, wherein the artificial RNA molecule regulates splicing of the pre-mRNA targeted by the (b).

**3.** The artificial RNA molecule of claim 1 or 2, wherein a base sequence of the (a) is the sequence set forth in SEQ ID NO: 2 or a sequence having a sequence identity of 80% or more with the sequence shown in SEQ ID NO: 2.

**4.** The artificial RNA molecule of claim 1 or 2, wherein a length of the (b) is 15 to 300 bases.

**5.** The artificial RNA molecule of claim 1 or 2, wherein the target sequence is an exon sequence.

**6.** The artificial RNA molecule of claim 1, comprising:
(c) a polynucleotide comprising a sequence that contributes to terminal stability,
wherein the (c) is arranged on the 3' side of the (b).

**7.** The artificial RNA molecule of claim 6, wherein the (c) is a polynucleotide comprising a transcription termination signal sequence recognized by an RNA pol III enzyme.

**8.** The artificial RNA molecule of claim 1 or 2, wherein the (a) is a polynucleotide binding to a protein that controls mRNA splicing.

**9.** The artificial RNA molecule of claim 1 or 2, wherein the target sequence is a partial sequence of a gene selected from an FAS gene, a dystrophin gene, and a fukutin gene.

**10.** The artificial RNA molecule of claim 1 or 2, wherein the target sequence is a partial sequence of an MAPT gene.

**11.** An expression vector comprising a DNA sequence encoding an artificial RNA molecule of claim 1 or 6.

**12.** A method of regulating mRNA splicing, the method comprising the steps of:

(A) preparing an artificial RNA molecule comprising:

(a) a polynucleotide potentially having a secondary structure represented by the formula (I), or a polynucleotide in which 1 to 3 bases are substituted, deleted, or added among 7 bases on the 3' side of the polynucleotide potentially having the secondary structure represented by the formula (I); and
(b) a pre-mRNA targeting polynucleotide comprising a sequence complementary to a target sequence that is a portion of a pe-mRNA;

wherein the (a) and the (b) are arranged from the 5' side to the 3' side in this order; and
(B) contacting the artificial RNA molecule prepared in the step (A) with a pre-mRNA to form a complementary strand between the artificial RNA molecule and the pre-mRNA, thereby regulating mRNA splicing.

**13.** The method of claim 12, wherein the regulation of mRNA splicing is caused by exon skipping.

**14.** The method of claim 12 or 13, wherein the base sequence of the (a) is the sequence set forth in SEQ ID NO: 2 or the sequence having the sequence identity of 80% or more with the sequence set forth in SEQ ID NO: 2.

**15.** The method of claim 12 or 13, wherein the target sequence is an exon sequence.

**16.** The method of claim 12 or 13, wherein the artificial RNA molecule comprises a polynucleotide comprising a transcription termination signal sequence recognized by an RNA pol III enzyme, the polynucleotide being arranged on the 3' side of the (b).

**17.** A method of producing a mature mRNA, the method comprising the steps of:

(A) preparing an artificial RNA molecule comprising:

(a) a polynucleotide potentially having a secondary structure represented by the formula (I), or a polynucleotide in which 1 to 3 bases are substituted, deleted, or added among 7 bases on the 3' side of the polynucleotide potentially having the secondary structure represented by the formula (I); and
(b) a pre-mRNA targeting polynucleotide comprising a sequence complementary to a target sequence that is a portion of a pre-mRNA;

wherein the (a) and the (b) are arranged from the 5' side to the 3' side in this order;
(B') contacting the artificial RNA molecule prepared in the step (A) with the pre-mRNA to form a complementary strand between the artificial RNA molecule and the pre-mRNA; and
(C) regulating mRNA splicing by the step (B') to produce a mature mRNA.

**18.** The method of claim 17, wherein the regulation of mRNA splicing is caused by exon skipping.

**19.** The method of claim 17 or 18, wherein the base sequence of the (a) is the sequence shown in SEQ ID NO: 2 or the sequence having the sequence identity of 80% or more with the sequence shown in SEQ ID NO: 2.

**20.** The method of claim 17 or 18, wherein the target sequence is an exon sequence.

**21.** The method of claim 17 or 18, wherein the artificial RNA molecule comprises a polynucleotide comprising a transcription termination signal sequence recognized by an RNA pol III enzyme, the polynucleotide being arranged on the 3' side of the (b).

FIG. 1

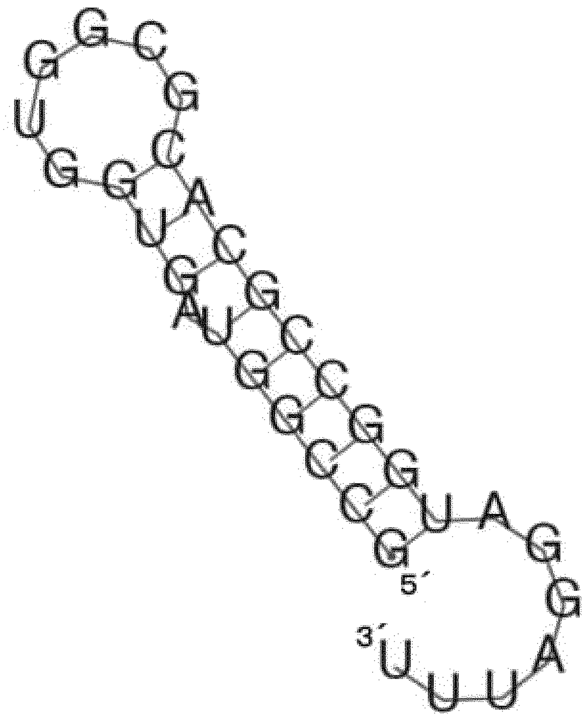


FIG. 2

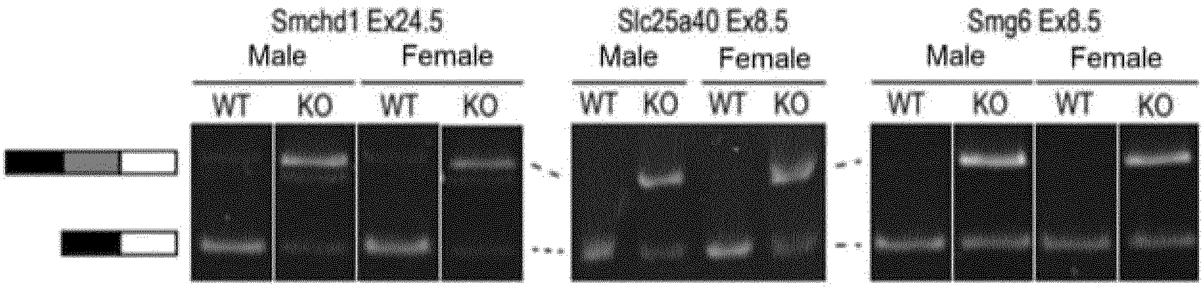


FIG. 3

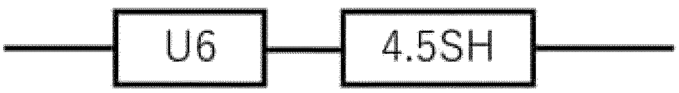

FIG.4

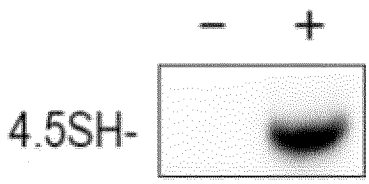


FIG.5

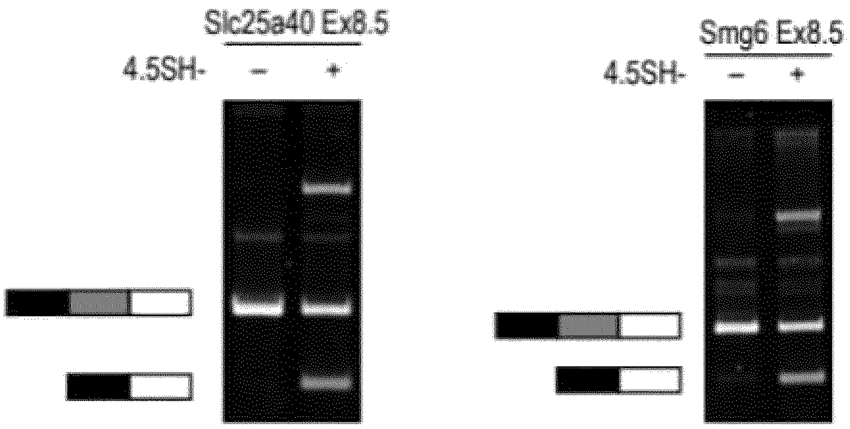


FIG.6

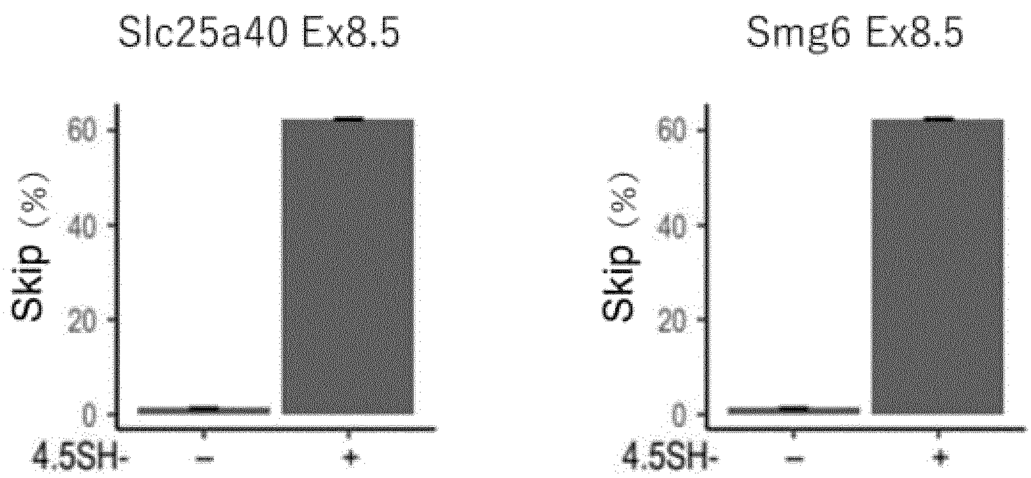

FIG. 7

4.5SH-WT 5´-...CCCTGGC...CCTACCTCTGCCTCC...-3´

Slc25a40 Ex8.5-WT 3´-...GGGTCCG...GGACGGAGACGGAGG...-5´

4.5SH-MUT 5´-...GGGTCCG...GGTAGGTGTCGGTGG...-3´

Slc25a40 Ex8.5-MUT 3´-...CCCTGGC...CCACCCACAGCCACC...-5´

FIG. 8

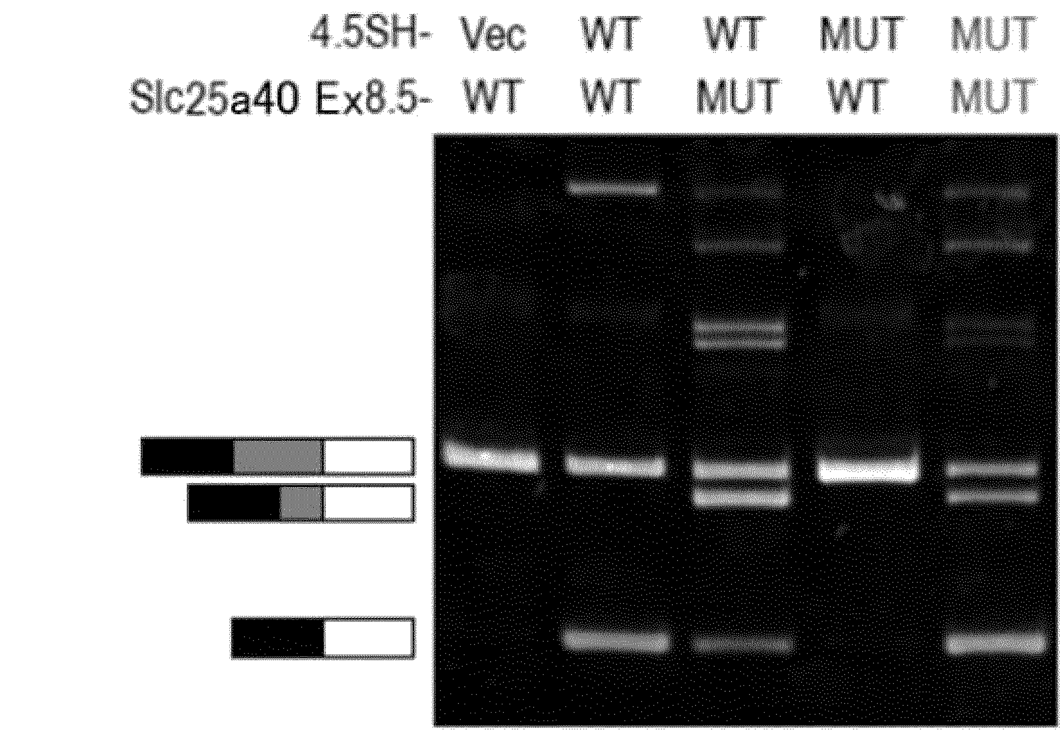

FIG.9

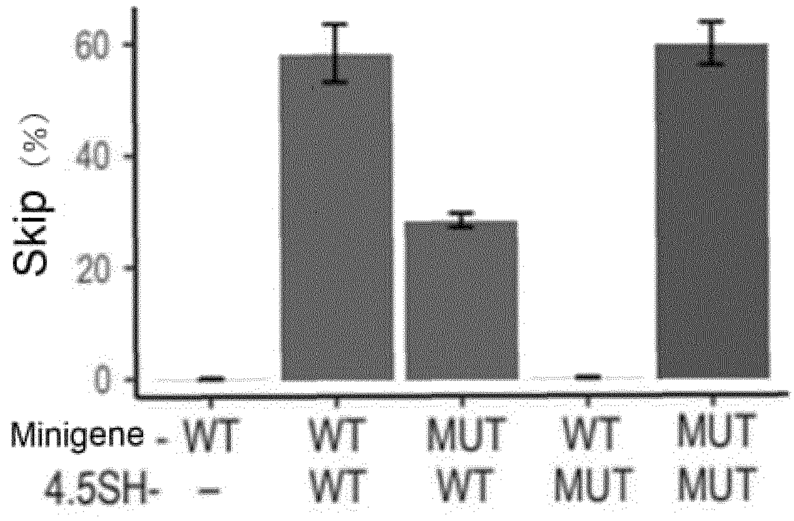
60
40
20
0
Skip (%)
Minigene - WT WT MUT WT MUT
4.5SH- - WT WT MUT MUT

FIG.10

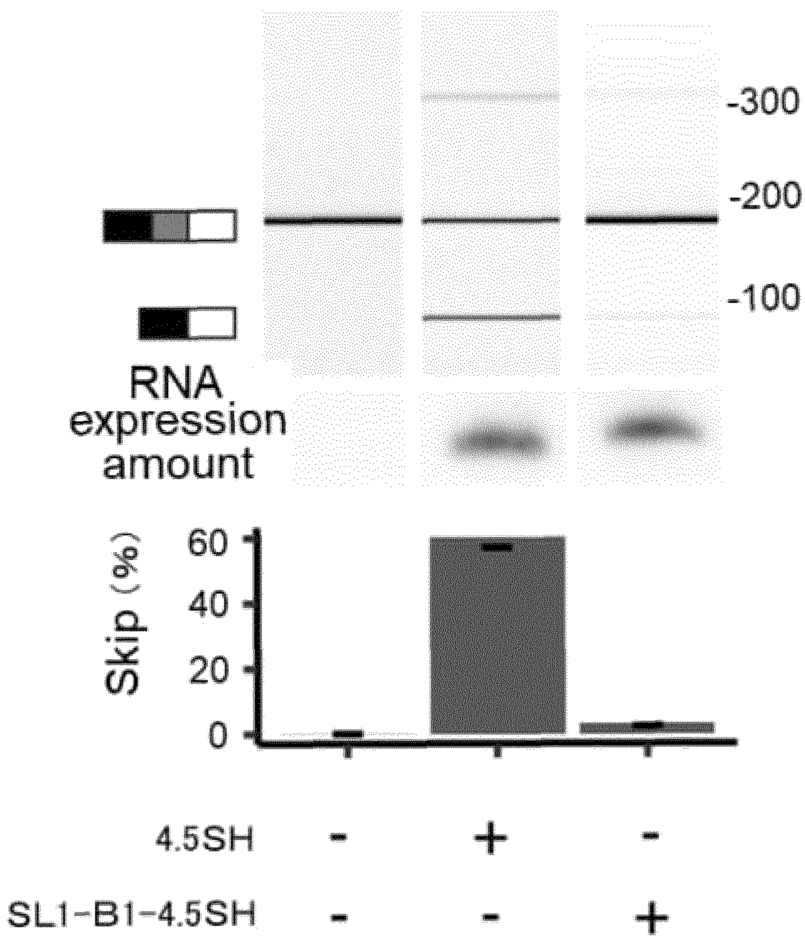
-300
-200
-100
RNA expression amount
60
40
20
0
Skip (%)
4.5SH - + -
SL1-B1-4.5SH - - +

FIG. 11

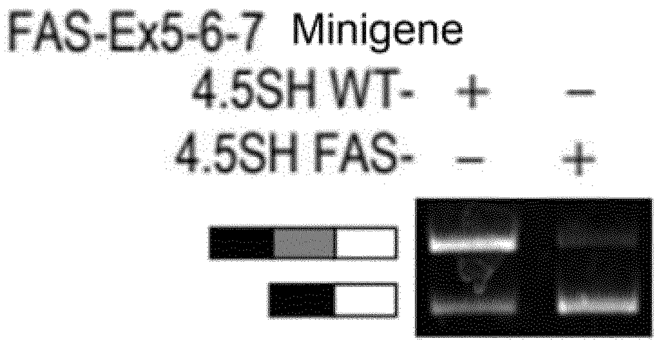


FIG. 12

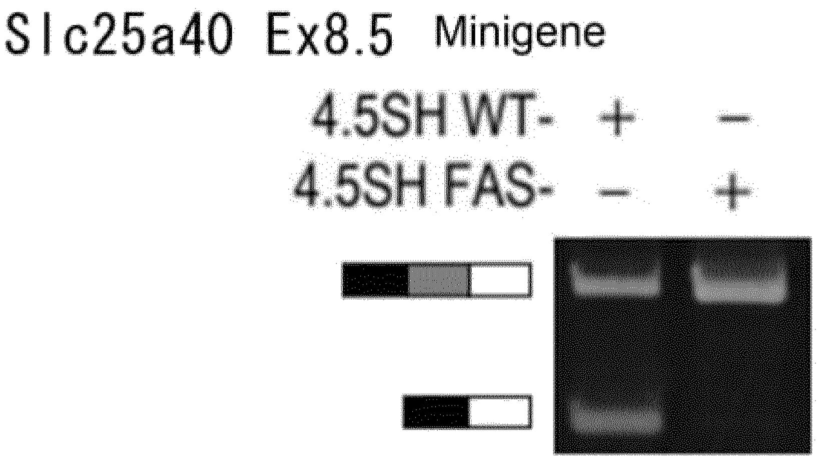


FIG. 13

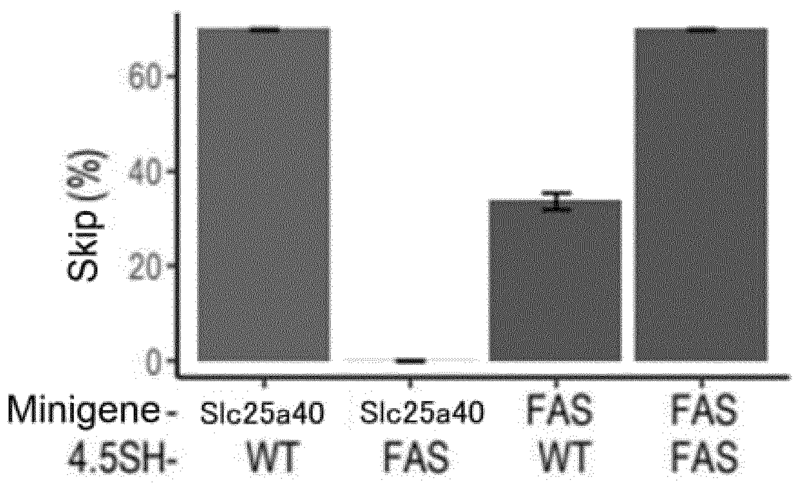

FIG. 14

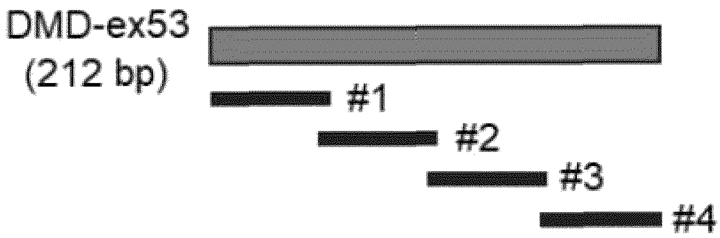


FIG. 15

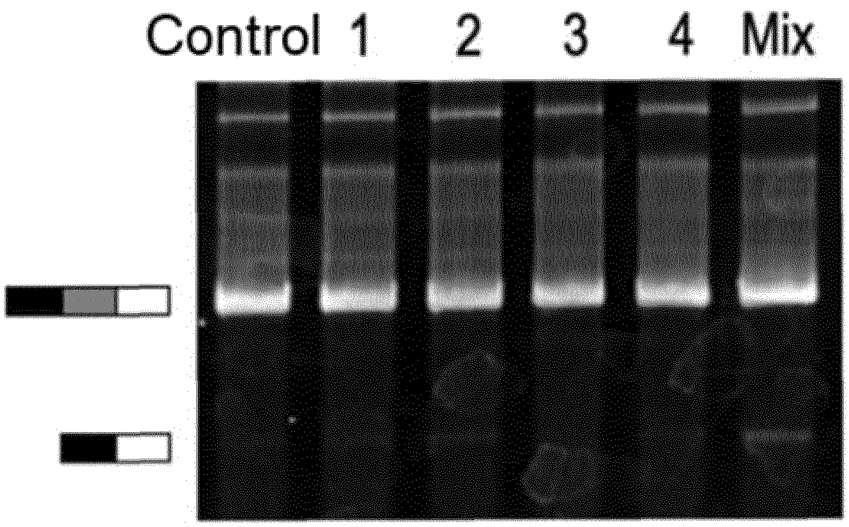


FIG. 16

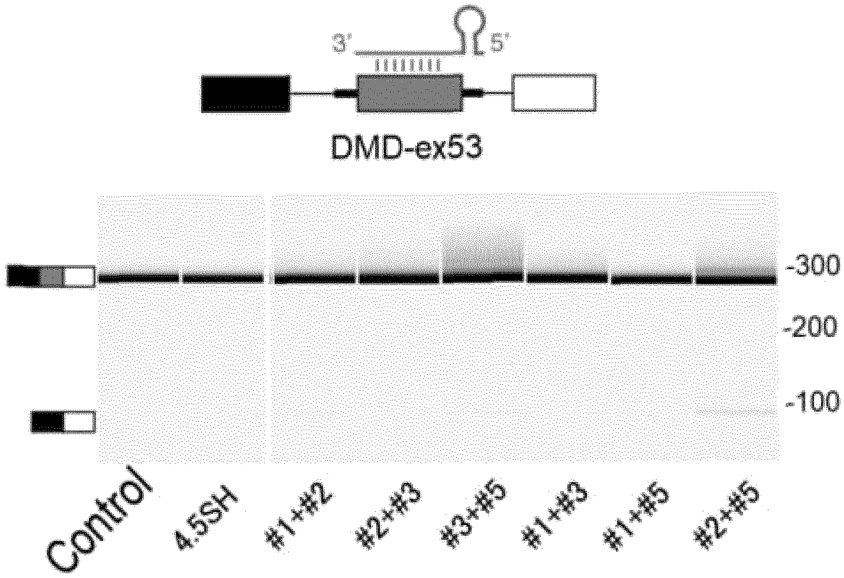

FIG. 17

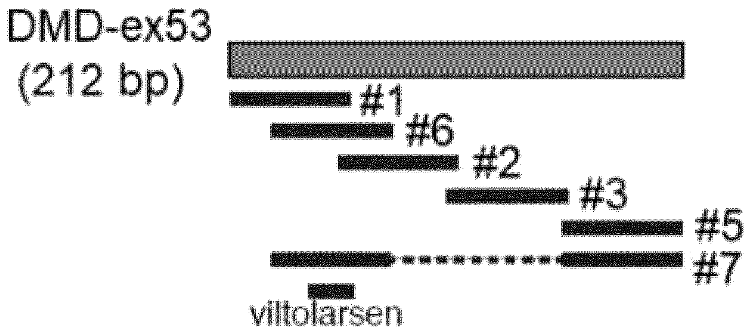
DMD-ex53
(212 bp)
#1
#6
#2
#3
#5
#7
viltolarsen

FIG. 18

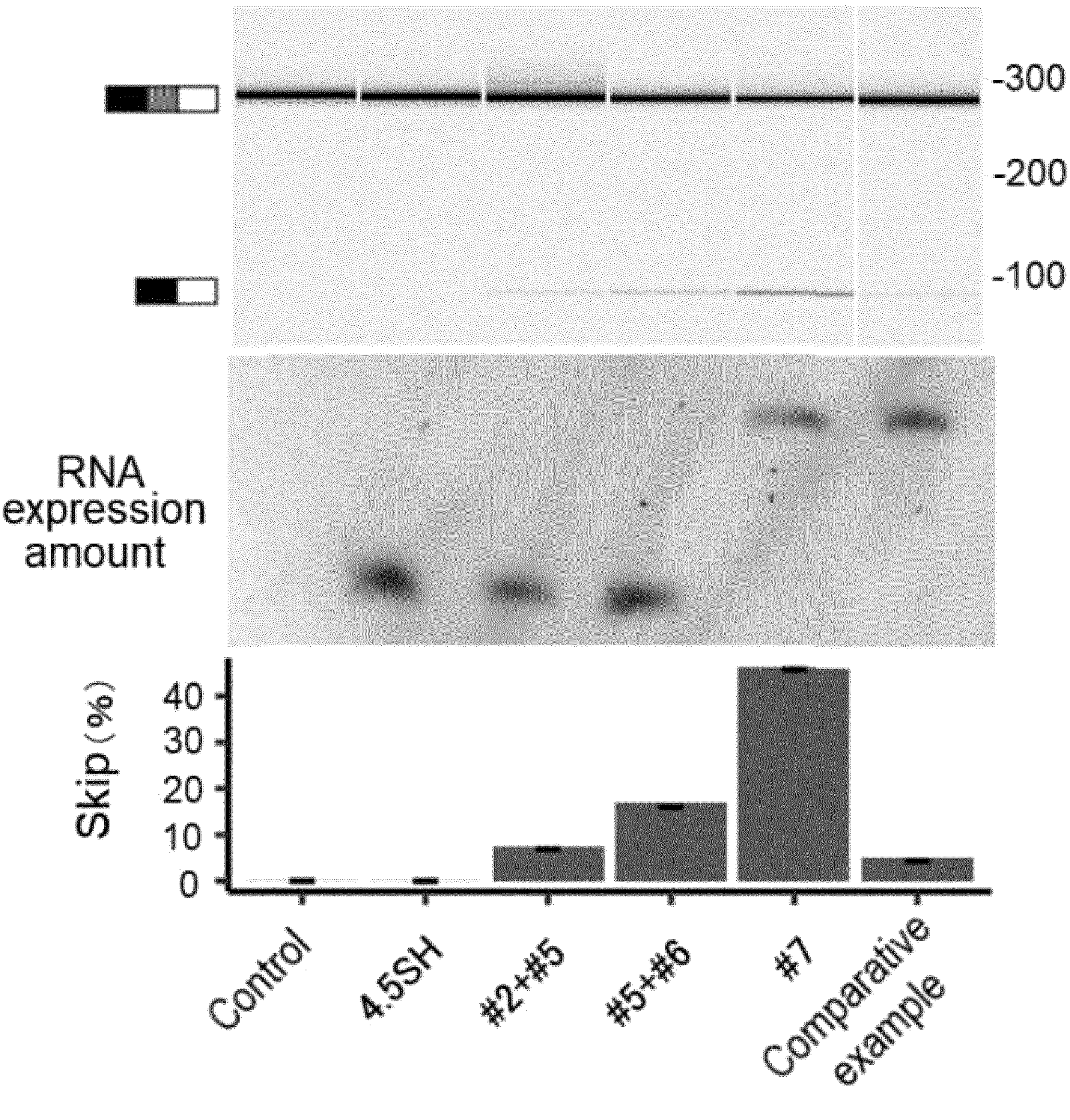
-300
-200
-100
RNA expression amount
Skip (%)
40
30
20
10
0
Control
4.5SH
#2+#5
#5+#6
#7
Comparative example

FIG. 19

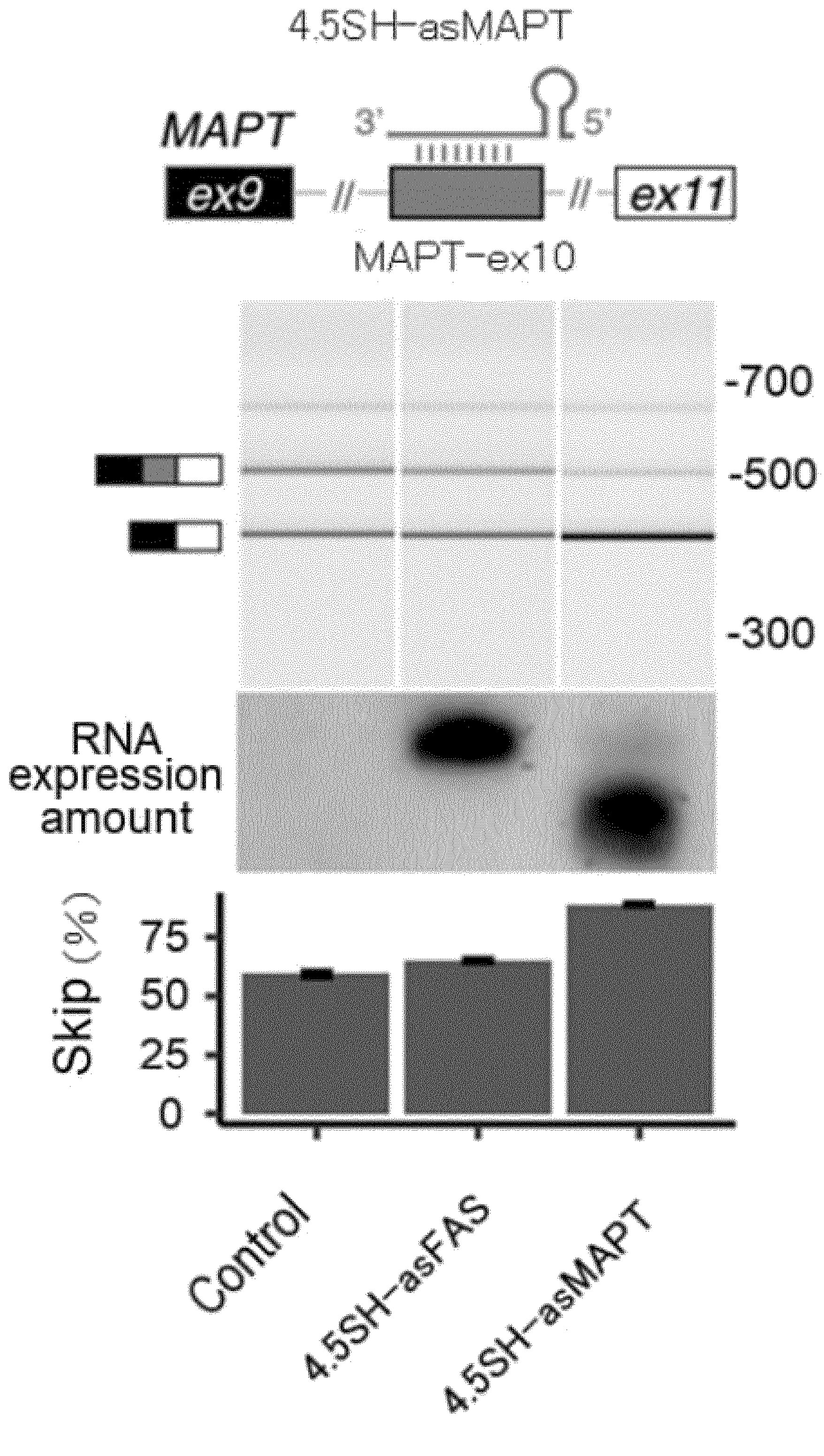
4.5SH-asMAPT
MAPT
3'
5'
ex9
ex11
MAPT-ex10
-700
-500
-300
RNA expression amount
Skip (%)
75
50
25
0
Control
4.5SH-asFAS
4.5SH-asMAPT

**INTERNATIONAL SEARCH REPORT**

International application No.
**PCT/JP2023/015828**

**A. CLASSIFICATION OF SUBJECT MATTER**

***C12N 15/113***(2010.01)i; ***C12N 15/10***(2006.01)i; ***C12N 15/63***(2006.01)i; ***C12P 19/34***(2006.01)i
FI: C12N15/113 Z ZNA; C12N15/63 Z; C12N15/10 Z; C12P19/34 A

According to International Patent Classification (IPC) or to both national classification and IPC

**B. FIELDS SEARCHED**

Minimum documentation searched (classification system followed by classification symbols)

C12N15/113; C12N15/10; C12N15/63; C12P19/34

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

CAplus/MEDLINE/EMBASE/BIOSIS (STN); GenBank/EMBL/DDBJ/GeneSeq

**C. DOCUMENTS CONSIDERED TO BE RELEVANT**

| Category* | Citation of document, with indication, where appropriate, of the relevant passages | Relevant to claim No. |
|---|---|---|
| A | WO 2021/117729 A1 (ASTELLAS PHARMA INC.) 17 June 2021 (2021-06-17) entire text | 1-21 |
| A | JP 2013-521791 A (ASSOCIATION INSTITUT DE MYOLOGIE) 13 June 2013 (2013-06-13) entire text | 1-21 |
| A | JP 2019-519218 A (VOYAGER THERAPEUTICS, INC.) 11 July 2019 (2019-07-11) entire text | 1-21 |
| A | ISHIDA, Kentaro et al. Regulation of gene expression via retrotransposon insertions and the noncoding RNA 4.5S RNAH. Genes to Cells. 03 September 2015, vol. 20, no. 11, pp. 887-901 abstract | 1-21 |
| P, X | NAKAGAWA, Shinichi et al. 4.5SH RNA counteracts deleterious exonization of SINE B1 in mice. Research Square. 02 September 2022, see https://www.researchsquare.com/article/rs-1949270/v1 (This document will not be sent. Refer to the URL if necessary.) entire text | 1-21 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **04 July 2023** | **11 July 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

INTERNATIONAL SEARCH REPORT

International application No.
PCT/JP2023/015828

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      ☑ in the form of an Annex C/ST.25 text file.
      ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      ☐ in the form of an Annex C/ST.25 text file (Rule 13*ter*.1(a)).
      ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

The "form of an Annex C/ST.25 text file" is replaced with the "form of ST.26."

Form PCT/ISA/210 (continuation of first sheet) (January 2015)

**INTERNATIONAL SEARCH REPORT**
**Information on patent family members**

International application No.
**PCT/JP2023/015828**

| Patent document cited in search report | Publication date (day/month/year) | Patent family member(s) | Publication date (day/month/year) |
|---|---|---|---|
| WO 2021/117729 A1 | 17 June 2021 | EP 4074825 A1<br>whole document<br>CN 114787357 A<br>KR 10-2022-0111322 A | |
| JP 2013-521791 A | 13 June 2013 | US 2013/0045538 A1<br>whole document<br>WO 2011/113889 A1<br>EP 2547768 A1 | |
| JP 2019-519218 A | 11 July 2019 | US 2019/0169616 A1<br>whole document<br>WO 2017/201248 A1<br>EP 3458588 A1<br>KR 10-2019-0005886 A<br>CN 110214187 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2014054250 A
- JP 2021166514 A


### Non-patent literature cited in the description


- **HARADA, F** ; **KATO, N**. *NAR.*, 1980, vol. 8, 1273-J285
- *Trends in Genetics*, December 2007, vol. 23, 614-622
- *Biochim Biophys Acta*, March 2013, vol. 1829 (3-4), 318-330
- **MOORE, M. J.** ; **P. A. SHARP**. *Nature*, 1993, vol. 365, 364-368
- **QINGMING YU et al.** *J Neurochem*, July 2004, vol. 90 (1), 164-72